# EUROPEAN PATENT SPECIFICATION

(11) **EP 3 055 423 B1**
(45) Date of publication and mention of the grant of the patent: **25.12.2019**
(21) Application number: 14783630.8
(22) Date of filing: 10.10.2014
(51) Int. Cl.: C12Q 1/6806, C12Q 1/6816, G01N 33/53

(54) **METHOD FOR DETECTING NUCLEIC ACID SEQUENCES OF INTEREST USING TALEN PROTEIN**
VERFAHREN ZUM NACHWEIS VON BESTIMMTEN NUKLEINSÄURESEQUENZEN ANHAND EINES TALEN PROTEINS
PROCÉDÉS DE DÉTECTION DE SÉQUENCES D'ACIDE NUCLÉIQUE D'INTÉRÊT À L'AIDE D'UN PROTEIN DU TYP TALEN

(30) Priority: 11.10.2013 DK 201370578
(43) Date of publication of application: 17.08.2016
(73) Proprietor: Cellectis, 75013 Paris (FR)
(72) Inventor: VALTON, Julien, New York, NY 10009 (US); JUILLERAT, Alexandre, New York, NY 10028 (US); DABOUSSI, Fayza, 31750 Escalquens (FR); BEURDELEY, Marine, 75009 Paris (FR); BERTONATI, Claudia, 68300 Saint Louis (FR); DUCHATEAU, Philippe, 91210 Draveil (FR)
(74) Representative: Santarelli
(86) International application number: PCT/EP2014/071812
(87) International publication number: WO 2015/052335

(56) References cited:
- EP-A1- 2 206 723
- WO-A1-2005/012575
- WO-A1-2011/094514
- WO-A1-2013/102289
- WO-A1-2013/102290
- WO-A2-01/62968
- WO-A2-2004/057016
- WO-A2-2011/096763
- WO-A2-2011/161420
- WO-A2-2014/018601
- US-A1- 2005 106 575
- US-A1- 2010 105 049
- US-A1- 2011 239 315
- US-A1- 2012 164 754
- DONG DENG ET AL: "Recognition of methylated DNA by TAL effectors", CELL RESEARCH, vol. 22, no. 10, 1 October 2012 (2012-10-01), pages 1502-1504, XP055084118, ISSN: 1001-0602, DOI: 10.1038/cr.2012.127
- PING YIN ET AL: "Specific DNA-RNA Hybrid Recognition by TAL Effectors", CELL REPORTS, vol. 2, no. 4, 1 October 2012 (2012-10-01) , pages 707-713, XP055075524, ISSN: 2211-1247, DOI: 10.1016/j.celrep.2012.09.001
- ROBERT MORBITZER ET AL: "Assembly of custom TALE-type DNA binding domains by modular cloning", NUCLEIC ACIDS RESEARCH, OXFORD : OXFORD UNIV. PRESS, 1974-ANFANGS: LONDON : INFORMATION RETRIEVAL LTD, GB, vol. 39, no. 13, 13 July 2011 (2011-07-13) , pages 5790-5799, XP002689132, ISSN: 0305-1048, DOI: 10.1093/NAR/GKR151 [retrieved on 2011-03-18]
- Joshua F. Meckler ET AL: "Quantitative analysis of TALE-DNA interactions suggests polarity effects", Nucleic Acids Research, vol. 41, no. 7, 1 April 2013 (2013-04-01), pages 4118-4128, XP55392937, ISSN: 0305-1048, DOI: 10.1093/nar/gkt085
- Hanhui Ma ET AL: "Visualization of repetitive DNA sequences in human chromosomes with transcription activator-like effectors", Proceedings National Academy of Sciences PNAS, vol. 110, no. 52, 24 December 2013 (2013-12-24), pages 21048-21053, XP55392927, US ISSN: 0027-8424, DOI: 10.1073/pnas.1319097110
- Moon-Soo Kim ET AL: "A zinc finger protein array for the visual detection of specific DNA sequences for diagnostic applications", Nucleic Acids Research, vol. 39, no. 5, 5 December 2010 (2010-12-05), pages e29-e29, XP55485358, UK ISSN: 0305-1048, DOI: 10.1093/nar/gkq1214

### SEQUENCE LISTING

<110> CELLECTIS
<120> METHODS FOR DETECTING NUCLEIC ACID SEQUENCES OF INTEREST USING
   DNA-BINDING PROTEIN DETECTOR
<130> P81313265PCT00
<160> 24
<170> PatentIn version 3.5
<210> 1
   <211> 1026
   <212> PRT
   <213> artificial sequence
<220>
   <223> RAGT2.4_TC_N152_C11_RenilaLuc (from pCLS 24992)
<400> 1
   Met Ser Pro Arg His His His His His His Ala Tyr Gly Glu Asn Leu
   1               5                   10                  15
   Tyr Phe Gln Gly Ala Met Ala Asp Leu Arg Thr Leu Gly Tyr Ser Gln
               20                  25                  30
   Gln Gln Gln Glu Lys Ile Lys Pro Lys Val Arg Ser Thr Val Ala Gln
           35                  40                  45
   His His Glu Ala Leu Val Gly His Gly Phe Thr His Ala His Ile Val
       50                  55                  60
   Ala Leu Ser Gln His Pro Ala Ala Leu Gly Thr Val Ala Val Lys Tyr
   65                  70                  75                  80
   Gln Asp Met Ile Ala Ala Leu Pro Glu Ala Thr His Glu Ala Ile Val
                   85                  90                  95
   Gly Val Gly Lys Gln Trp Ser Gly Ala Arg Ala Leu Glu Ala Leu Leu
               100                 105                 110
   Thr Val Ala Gly Glu Leu Arg Gly Pro Pro Leu Gln Leu Asp Thr Gly
           115                 120                 125
   Gln Leu Leu Lys Ile Ala Lys Arg Gly Gly Val Thr Ala Val Glu Ala
       130                 135                 140
   Val His Ala Trp Arg Asn Ala Leu Thr Gly Ala Pro Leu Asn Leu Thr
   145                 150                 155                 160
   Pro Gln Gln Val Val Ala Ile Ala Ser Asn Asn Gly Gly Lys Gln Ala
                   165                 170                 175
   Leu Glu Thr Val Gln Arg Leu Leu Pro Val Leu Cys Gln Ala His Gly
               180                 185                 190
   Leu Thr Pro Gln Gln Val Val Ala Ile Ala Ser Asn Gly Gly Gly Lys
           195                 200                 205
   Gln Ala Leu Glu Thr Val Gln Arg Leu Leu Pro Val Leu Cys Gln Ala
       210                 215                 220
   His Gly Leu Thr Pro Gln Gln Val Val Ala Ile Ala Ser Asn Gly Gly
   225                 230                 235                 240
   Gly Lys Gln Ala Leu Glu Thr Val Gln Arg Leu Leu Pro Val Leu Cys
                   245                 250                 255
   Gln Ala His Gly Leu Thr Pro Gln Gln Val Val Ala Ile Ala Ser Asn
               260                 265                 270
   Gly Gly Gly Lys Gln Ala Leu Glu Thr Val Gln Arg Leu Leu Pro Val
           275                 280                 285
   Leu Cys Gln Ala His Gly Leu Thr Pro Glu Gln Val Val Ala Ile Ala
       290                 295                 300
   Ser Asn Ile Gly Gly Lys Gln Ala Leu Glu Thr Val Gln Ala Leu Leu
   305                 310                 315                 320
   Pro Val Leu Cys Gln Ala His Gly Leu Thr Pro Gln Gln Val Val Ala
                   325                 330                 335
   Ile Ala Ser Asn Gly Gly Gly Lys Gln Ala Leu Glu Thr Val Gln Arg
               340                 345                 350
   Leu Leu Pro Val Leu Cys Gln Ala His Gly Leu Thr Pro Gln Gln Val
           355                 360                 365
   Val Ala Ile Ala Ser Asn Asn Gly Gly Lys Gln Ala Leu Glu Thr Val
       370                 375                 380
   Gln Arg Leu Leu Pro Val Leu Cys Gln Ala His Gly Leu Thr Pro Gln
   385                 390                 395                 400
   Gln Val Val Ala Ile Ala Ser Asn Asn Gly Gly Lys Gln Ala Leu Glu
                   405                 410                 415
   Thr Val Gln Arg Leu Leu Pro Val Leu Cys Gln Ala His Gly Leu Thr
               420                 425                 430
   Pro Gln Gln Val Val Ala Ile Ala Ser Asn Gly Gly Gly Lys Gln Ala
           435                 440                 445
   Leu Glu Thr Val Gln Arg Leu Leu Pro Val Leu Cys Gln Ala His Gly
       450                 455                 460
   Leu Thr Pro Gln Gln Val Val Ala Ile Ala Ser Asn Gly Gly Gly Lys
   465                 470                 475                 480
   Gln Ala Leu Glu Thr Val Gln Arg Leu Leu Pro Val Leu Cys Gln Ala
                   485                 490                 495
   His Gly Leu Thr Pro Glu Gln Val Val Ala Ile Ala Ser Asn Ile Gly
               500                 505                 510
   Gly Lys Gln Ala Leu Glu Thr Val Gln Ala Leu Leu Pro Val Leu Cys
           515                 520                 525
   Gln Ala His Gly Leu Thr Pro Glu Gln Val Val Ala Ile Ala Ser His
       530                 535                 540
   Asp Gly Gly Lys Gln Ala Leu Glu Thr Val Gln Arg Leu Leu Pro Val
   545                 550                 555                 560
   Leu Cys Gln Ala His Gly Leu Thr Pro Gln Gln Val Val Ala Ile Ala
                   565                 570                 575
   Ser Asn Gly Gly Gly Lys Gln Ala Leu Glu Thr Val Gln Arg Leu Leu
               580                 585                 590
   Pro Val Leu Cys Gln Ala His Gly Leu Thr Pro Gln Gln Val Val Ala
           595                 600                 605
   Ile Ala Ser Asn Gly Gly Gly Lys Gln Ala Leu Glu Thr Val Gln Arg
       610                 615                 620
   Leu Leu Pro Val Leu Cys Gln Ala His Gly Leu Thr Pro Glu Gln Val
   625                 630                 635                 640
   Val Ala Ile Ala Ser Asn Ile Gly Gly Lys Gln Ala Leu Glu Thr Val
                   645                 650                 655
   Gln Ala Leu Leu Pro Val Leu Cys Gln Ala His Gly Leu Thr Pro Gln
               660                 665                 670
   Gln Val Val Ala Ile Ala Ser Asn Gly Gly Gly Arg Pro Ala Leu Glu
           675                 680                 685
   Ile Arg Ser Arg Ala Ser Arg Ser Ile Val Ala Gln Leu Ser Arg Pro
       690                 695                 700
   Asp Pro Ser Gly Ser Gly Ser Gly Gly Asp Pro Met Thr Ser Lys Val
   705                 710                 715                 720
   Tyr Asp Pro Glu Gln Arg Lys Arg Met Ile Thr Gly Pro Gln Trp Trp
                   725                 730                 735
   Ala Arg Cys Lys Gln Met Asn Val Leu Asp Ser Phe Ile Asn Tyr Tyr
               740                 745                 750
   Asp Ser Glu Lys His Ala Glu Asn Ala Val Ile Phe Leu His Gly Asn
           755                 760                 765
   Ala Ala Ser Ser Tyr Leu Trp Arg His Val Val Pro His Ile Glu Pro
       770                 775                 780
   Val Ala Arg Cys Ile Ile Pro Asp Leu Ile Gly Met Gly Lys Ser Gly
   785                 790                 795                 800
   Lys Ser Gly Asn Gly Ser Tyr Arg Leu Leu Asp His Tyr Lys Tyr Leu
                   805                 810                 815
   Thr Ala Trp Phe Glu Leu Leu Asn Leu Pro Lys Lys Ile Ile Phe Val
               820                 825                 830
   Gly His Asp Trp Gly Ala Cys Leu Ala Phe His Tyr Ser Tyr Glu His
           835                 840                 845
   Gln Asp Lys Ile Lys Ala Ile Val His Ala Glu Ser Val Val Asp Val
       850                 855                 860
   Ile Glu Ser Trp Asp Glu Trp Pro Asp Ile Glu Glu Asp Ile Ala Leu
   865                 870                 875                 880
   Ile Lys Ser Glu Glu Gly Glu Lys Met Val Leu Glu Asn Asn Phe Phe
                   885                 890                 895
   Val Glu Thr Met Leu Pro Ser Lys Ile Met Arg Lys Leu Glu Pro Glu
               900                 905                 910
   Glu Phe Ala Ala Tyr Leu Glu Pro Phe Lys Glu Lys Gly Glu Val Arg
           915                 920                 925
   Arg Pro Thr Leu Ser Trp Pro Arg Glu Ile Pro Leu Val Lys Gly Gly
       930                 935                 940
   Lys Pro Asp Val Val Gln Ile Val Arg Asn Tyr Asn Ala Tyr Leu Arg
   945                 950                 955                 960
   Ala Ser Asp Asp Leu Pro Lys Met Phe Ile Glu Ser Asp Pro Gly Phe
                   965                 970                 975
   Phe Ser Asn Ala Ile Val Glu Gly Ala Lys Lys Phe Pro Asn Thr Glu
               980                 985                 990
   Phe Val Lys Val Lys Gly Leu His Phe Ser Gln Glu Asp Ala Pro Asp
           995                 1000                1005
   Glu Met Gly Lys Tyr Ile Lys Ser Phe Val Glu Arg Val Leu Lys Asn
       1010                1015                1020
   Glu Gln
   1025
<210> 2
   <211> 119
   <212> DNA
   <213> artificial sequence
<220>
   <223> Avr15RAGT2R_Forward
<400> 2
   gtacaaaaaa gcagtcgact agggataaca gggtaattat ataaacctaa ccctcttagc     60
   atgaaggtac cataagtaac cataaacaat gcatttactt ataatacagt ttagctttc     119
<210> 3
   <211> 119
   <212> DNA
   <213> artificial sequence
<220>
   <223> Avr15RAGT2R_Reverse
<400> 3
   gaaagctaaa ctgtattata agtaaatgca ttgtttatgg ttacttatgg taccttcatg     60
   ctaagagggt taggtttata taattaccct gttatcccta gtcgactgct tttttgtac     119
<210> 4
   <211> 91
   <212> DNA
   <213> artificial sequence
<220>
   <223> Avr15RAGT2R_FOR_5Biot
<400> 4
   gtacaaaaaa gcagtcgact agggataaca gggtaattat ataaacctaa ccctcttagc     60
   atgaaggtac cataagtaac cataaacaat g                                    91
<210> 5
   <211> 22
   <212> DNA
   <213> artificial sequence
<220>
   <223> Avr15RAGT2R_Pray_REV1
<400> 5
   cattgtttat ggttacttat gg 22
<210> 6
   <211> 22
   <212> DNA
   <213> artificial sequence
<220>
   <223> Avr15RAGT2R_Pray_REV2
<400> 6
   catggtttat ggttacttat gg 22
<210> 7
   <211> 22
   <212> DNA
   <213> artificial sequence
<220>
   <223> Avr15RAGT2R_Pray_REV3
<400> 7
   catggcttat ggttacttat gg 22
<210> 8
   <211> 22
   <212> DNA
   <213> artificial sequence
<220>
   <223> Avr15RAGT2R_Pray_REV4
<400> 8
   catggctttt ggttacttat gg 22
<210> 9
   <211> 22
   <212> DNA
   <213> artificial sequence
<220>
   <223> Avr15RAGT2R_Pray_REV5
<400> 9
   catggctttt gcttacttat gg 22
<210> 10
   <211> 22
   <212> DNA
   <213> artificial sequence
<220>
   <223> Avr15RAGT2R_Pray_REV6
<400> 10
   gctggcactt gcttgctaca gc 22
<210> 11
   <211> 22
   <212> DNA
   <213> artificial sequence
<220>
   <223> Avr15RAGT2R_Pray_REV7
<400> 11
   cattgtttat ggttacttac gg 22
<210> 12
   <211> 22
   <212> DNA
   <213> artificial sequence
<220>
   <223> Avr15RAGT2R_Pray_REV8
<400> 12
   cattgtttat ggttacctat gg 22
<210> 13
   <211> 22
   <212> DNA
   <213> artificial sequence
<220>
   <223> Avr15RAGT2R_Pray_REV9
<400> 13
   cattgtttat ggctacttat gg 22
<210> 14
   <211> 22
   <212> DNA
   <213> artificial sequence
<220>
   <223> Avr15RAGT2R_Pray_REV10
<400> 14
   cattgtttac ggttacttat gg 22
<210> 15
   <211> 22
   <212> DNA
   <213> artificial sequence
<220>
   <223> Avr15RAGT2R_Pray_REV11
<400> 15
   cattgtctat ggttacttat gg 22
<210> 16
   <211> 22
   <212> DNA
   <213> artificial sequence
<220>
   <223> Avr15RAGT2R_Pray_REV12
<400> 16
   catcgtttat ggttacttat gg 22
<210> 17
   <211> 22
   <212> DNA
   <213> artificial sequence
<220>
   <223> Avr15RAGT2R_Pray_REV13
<400> 17
   cattgtttat ggctacctac gg 22
<210> 18
   <211> 22
   <212> DNA
   <213> artificial sequence
<220>
   <223> Avr15RAGT2R_Pray_REV14
<400> 18
   catcgtctac ggttacttat gg 22
<210> 19
   <211> 22
   <212> DNA
   <213> artificial sequence
<220>
   <223> Avr15RAGT2R_Pray_REV15
<400> 19
   catcgtctat ggttacttat gg 22
<210> 20
   <211> 22
   <212> DNA
   <213> artificial sequence
<220>
   <223> Avr15RAGT2R_Pray_REV16
<400> 20
   cattgtttat ggttacctac gg 22
<210> 21
   <211> 91
   <212> DNA
   <213> artificial sequence
<220>
   <223> Avr15RAGT2R_REV_3Biot
<400> 21
   cattgtttat ggttacttat ggtaccttca tgctaagagg gttaggttta tataattacc 60
   ctgttatccc tagtcgactg cttttttgta c 91
<210> 22
   <211> 22
   <212> DNA
   <213> artificial sequence
<220>
   <223> Avr15RAGT2R_Pray_For1
<400> 22
   ccataagtaa ccataaacaa tg 22
<210> 23
   <211> 22
   <212> RNA
   <213> artificial sequence
<220>
   <223> Avr15RAGT2R_Pray_RNA_For1
<400> 23
   ccauaaguaa ccauaaacaa ug 22
<210> 24
   <211> 22
   <212> RNA
   <213> artificial sequence
<220>
   <223> Avr15RAGT2R_Pray_RNA_REV6
<400> 24
   gcuggcacuu gcuugcuaca gc 22

## Description

### Field of the invention

The present invention relates to a method for the detection of a specific nucleic acid in a sample or in a cell. Specifically, the invention provides a method for detecting the presence of a specific nucleic acid using engineered DNA-binding domains derived from Transcription Activator Like-Effectors (TALE). The method of the invention is particularly useful for *in vitro* diagnostic application.

### Background of the invention

Transcription activator-like effectors (TALEs), a group of bacterial plant pathogen proteins, have emerged as new engineerable scaffolds for production of tailored DNA binding domains with chosen specificities. Interest in these systems comes from the apparent simple cipher governing DNA recognition by their DNA binding domain (Boch, Scholze et al. 2009; Moscou and Bogdanove 2009). The TALE DNA-binding domain is composed of multiple TALE repeats that individually recognize one DNA base pair through specific amino acid di-residues (repeat variable di-residues or RVDs). The remarkably high specificity of TALE repeats and the apparent absence of context dependence effects among repeats in an array, allow modular assembly of TALE DNA binding domains able to recognize almost any DNA sequence of interest. Within the past two years, engineered TALE DNA binding domains have been fused to transcription activator (dTALEs) (Morbitzer, Elsaesser et al. 2011), repressor (Cong, Zhou et al. 2012) or nuclease domains (TALENs) (Christian, Cermak et al. 2010) and used to specifically regulate or modify genes of interest (Bogdanove and Voytas 2011). Recently, TALE DNA binding domains have been also used to specifically target a native section of a chromosome for purification and proteomic analysis (Byrum, Taverna et al. 2013). More recently, further modular base per base nucleic acid binding domain referred as MBBBD have been discovered with a similar ability to bind 10 to 20 base pairs nucleic acid target sequence upon expression into cells (WO2014/018601).

The detection of specific nucleic acid sequence has been largely used for a wide range of applications including as non limiting examples in vitro diagnostic, indication of the presence of an organism, research in the field of molecular biology. In particular, detection of a specific nucleic acid sequence in a physiological sample constitutes, at the present time, the major line of development of diagnostic methods, e.g. for identifying the probability of bacteria of developing antibiotic resistance, genetic abnormalities, the risks of cancer associated with genetic modifications and viral infections.

A number of procedures are presently available for the detection of specific nucleic acid molecules. Several techniques have been developed over the years, all relying on the detection of a hybrid molecule between the target nucleic acid molecule and a specific labeled probe such as *in situ* hybridization which allows the detection and localization in a cell of a specific DNA sequence using labeled probes.

Today, the most widely used methods to detect nucleic acids are based on the polymerase chain reaction (PCR). Real-time PCR for example is used to amplify and simultaneously quantify a targeted DNA molecule. As well as PCR, a variety of other techniques have been developed for detection and amplification of specific sequences. For direct detection, the target nucleic acid is most commonly separated on the basis of size by gel electrophoresis and transferred to a solid support prior to hybridization with a probe complementary to the target sequence (Southern and Northern blotting). Another detection method is based on engineered zinc finger proteins wherein the signal transducer is based on the binding dependent reassembly of a reporter protein, such that no signal should be present unless DNA binding occurs, but the sensitivity of this method need to be improved in order to be used to detect unique sequences in cells (Ghosh, Stains et al, molecular biosystems 2006; Kim, Segal et al. 2011, NAR).

However, all the methods developed so far suffer from serious drawbacks. In particular, amplification of the target sequence is time consuming, increases the probability of errors, and is highly prone to contamination. Moreover, they all make use of labeled nucleotides, thus contributing to seriously increasing the overall costs. To overcome these drawbacks, the inventors sought to develop a rapid test using TALE engineered DNA binding domain to detect a specific nucleic acid sequence. They develop a low-cost method, simple to operate and which is adapted for use in low resource settings.

Moon-Soo Kim ET AL: "A zinc finger protein array for the visual detection of specific DNA sequences for diagnostic applications", Nucleic Acids Research, vol. 39(5), page e29, discloses that ZFP may be used in visual detection of double-stranded DNA sequences in diagnostic samples and mentions that TAL effector DNA binding domains might also have characteristics favorable for this purpose.

Ping Yin ETAL: "Specific DNA-RNA Hybrid Recognition by TAL Effectors", CELL REPORTS, vol. 2(4), 2012, pages 707-713, discloses that an engineered TALE can bind to a HIV-derived DNA-RNA hybrid and thereby protect the RNA from degradation, thus preventing replication of the retroviruses. Ping Yin ET AL deduce that TAL effectors could be used in antiviral therapies.

### Summary of the invention

The present invention relates to a new rapid diagnostic tool based on TALE-protein. The inventors develop a method of detecting a nucleic acid sequence of interest, as defined in the claims, comprising: providing nucleic acids which can comprise a nucleic acid sequence of interest; providing at least one DNA binding domain capable of binding said specific nucleic acid sequence; contacting nucleic acids with said DNA binding domain; removing unbound DNA-binding domain and nucleic acid and detecting the presence or the absence of said nucleic acid sequence of interest. Said nucleic acid is a single strand nucleic acid. The single strand nucleic acid is detected once hybridized with a complementary sequence. One or more DNA binding domains which bind each different part of the nucleic acid sequence of interest can be used in this method. This method can be realized in solution with or without the help of a solid support. Different detection techniques can be used, included as non limiting example direct protein detection (e;g. immunohistochemistry, immunoassay), detection of reporter molecule fused to DNA binding domain or labeled nucleic acid sequence (e. g. fluorescent protein, tag, chemiluminescent proteins, enzyme), nucleic acid detection (e.g. PCR, hybridization) or indirect detection by comparing the result with standard sample comprising known amounts of a purified nucleic acid or protein (e. g. bovine serum albumin (BSA)), by a competitive protein binding assay.

### Brief description of the figures

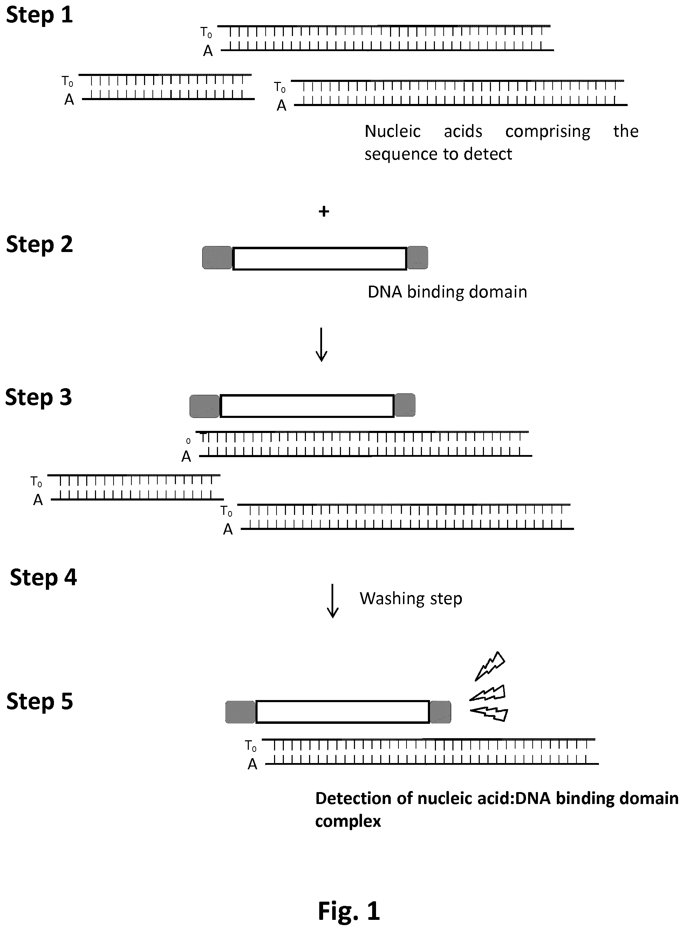

**Figure 1****:** A schematic example of the DNA binding domain-based nucleic acid detection method. Among a pool of nucleic acids (step 1), a nucleic acid sequence of interest is detected by providing a DNA binding domain capable of binding the nucleic acid sequence of interest (step 2) and contacting the DNA binding domain with nucleic acids (step 3). After removing the unbound DNA binding domains and nucleic acids (step 4), DNA binding domain: nucleic acid complex is detected by methods well known in the art (step 5).

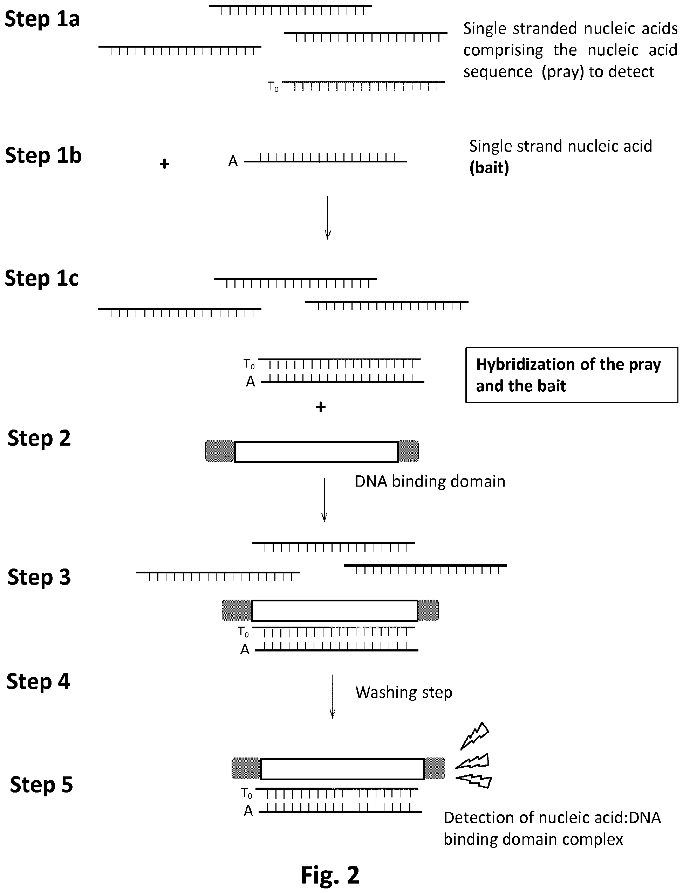

**Figure 2****:** A schematic example of single strand pray sequence detection method. To detect the single strand pray sequence among nucleic acids, a single strand nucleic acid (bait) comprising a complementary sequence to pray sequence is provided (step 1b). Among nucleic acids, only the specific pray sequence hybridized with the bait sequence under suitable conditions (step 1c). Then, a DNA binding domain capable of specifically binding the hybridized pray-bait sequence is provided (step 2). After contacting DNA binding domain with hybridized nucleic acid (step 3) and removing the unbound DNA binding domains and nucleic acids (step 4), the DNA binding domain:pray:bait complex is detected using methods well known in the art (step 5).

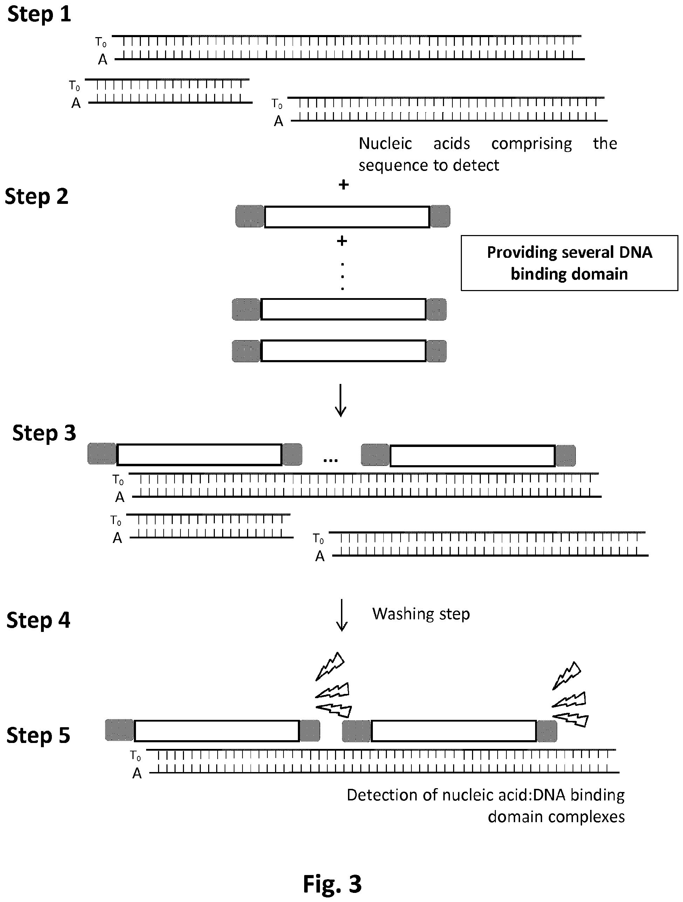

**Figure 3****:** A schematic example of the multiplex nucleic acid detection methods. Among nucleic acids (step 1), a nucleic acid sequence of interest is detected by providing (step 2) several DNA binding domains capable of binding different parts of the nucleic acid sequence of interest with nucleic acids (step 3). After contacting DNA binding domains with nucleic acids and removing the unbound DNA binding domains and nucleic acids (step 4), DNA binding domain: nucleic acid complexes are detected by methods well known in the art (step 5).

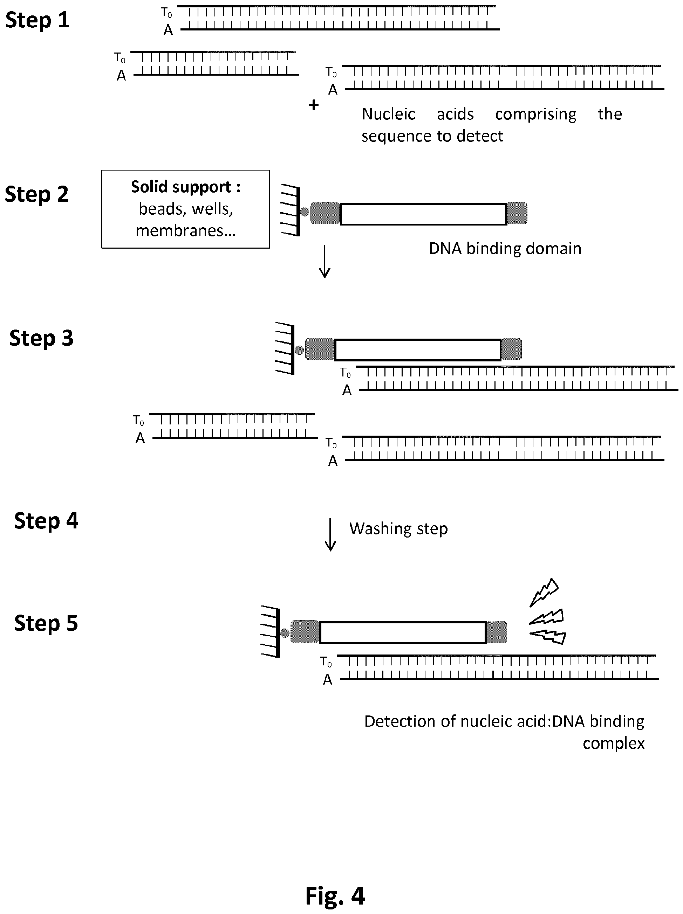

**Figure 4****:** A schematic example of nucleic acid detection method on solid support. Among nucleic acids (step 1), a nucleic acid sequence of interest is detected by providing a DNA binding domain capable of binding the nucleic acid sequence of interest. The DNA binding domain is immobilized on a solid support (step 2) to capture the nucleic acid sequence of interest (step 3). After removing the unbound DNA binding domains and nucleic acids (step 4), the DNA binding domain: nucleic acid complex is detected by methods well known in the art (step 5).

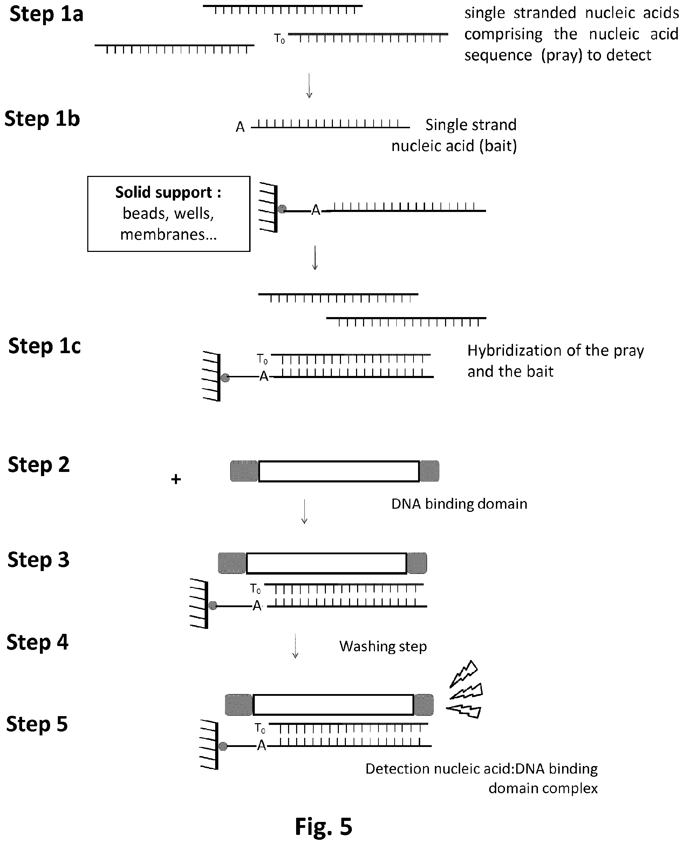

**Figure 5****:** A schematic example of a single strand nucleic acid detection method on solid support. Among nucleic acids (step 1), a nucleic acid sequence of interest (pray) is detected by providing a bait sequence immobilized on a solid support. Among nucleic acids, only the specific pray sequence hybridized to bait sequence under suitable conditions (step 1c). Then, a DNA binding domain capable of binding the hybridized sequence is provided (step2). After contacting the DNA binding domain with hybridized nucleic acid (step 3) and removing the unbound DNA binding domains and nucleic acids (step 4), the DNA binding domain:pray:bait complex is detected by methods well known in the art (step 5).

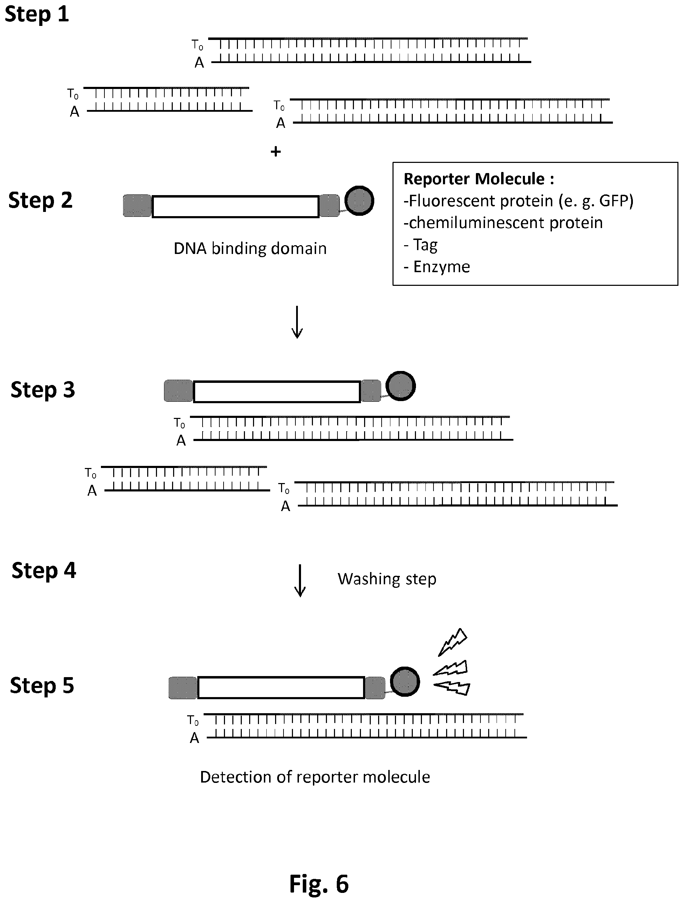

**Figure 6****:** A schematic example of nucleic acid detection method using reporter molecule. Among nucleic acids (step 1), a nucleic acid sequence of interest is detected by providing a specific DNA binding domain fused to a reporter molecule (step2). After contacting the DNA binding domain with nucleic acids (step 3) and removing the unbound DNA binding domains and nucleic acids (step 4), the reporter molecule is detected by methods well known in the art (step 5).

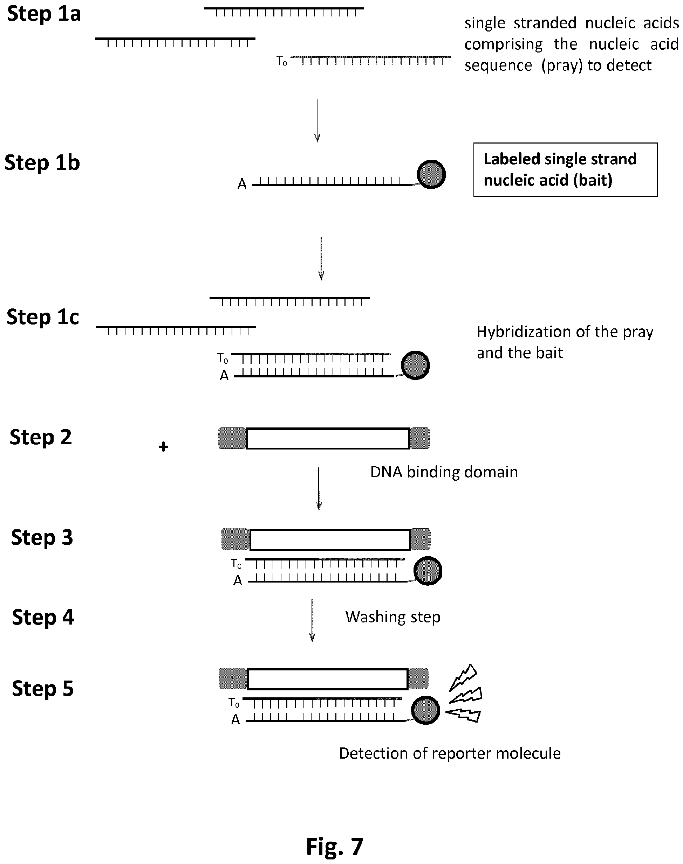

**Figure 7****:** A schematic example of single strand nucleic acid detection method using labeled bait. To detect the single strand pray sequence among nucleic acids, a labeled single strand nucleic acid (bait) comprising a complementary sequence to pray sequence is provided (step 1b). Only the specific pray sequence hybridized to the bait sequence under suitable conditions (step 1c). Then, to detect pray:bait sequence, a DNA binding domain capable of specifically binding the hybridized pray-bait sequence is provided (step 2). After contacting the DNA binding domain with nucleic acids (step 3) and removing the unbound DNA binding domains and nucleic acids (step 4), the labeled nucleic acid is detected using methods well known in the art (step 5).

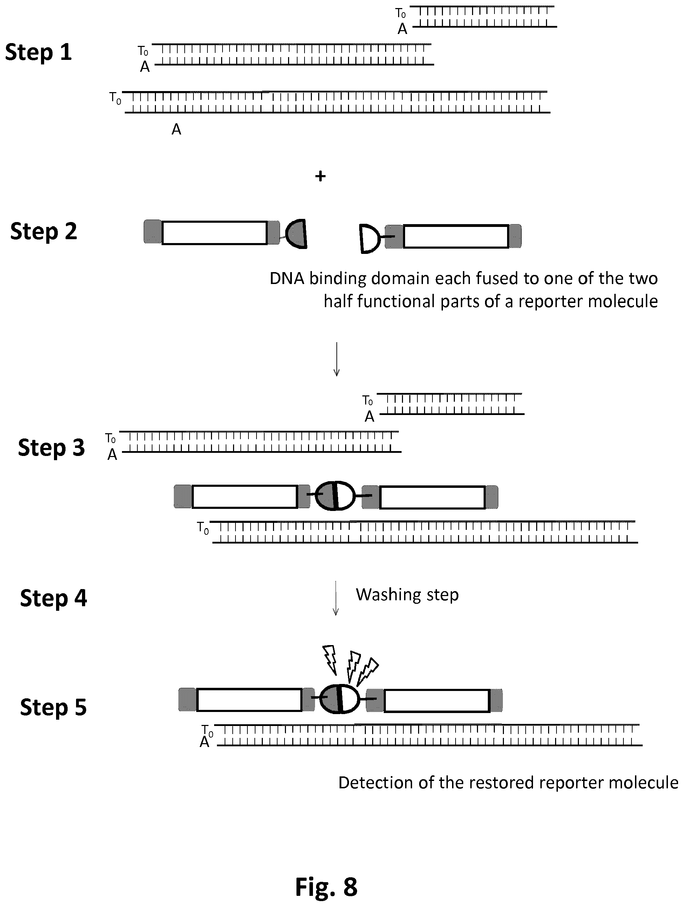

**Figure 8****:** A schematic example of nucleic acid detection method using protein complementation assay. Among nucleic acids (step 1), a nucleic acid sequence of interest is detected by providing two specific DNA binding domains each fused to one of the two half functional domain of a reporter molecule (step 2). After contacting the DNA binding domains with nucleic acids (step 3) and removing the unbound DNA binding domains and nucleic acids (step 4), the restored reporter molecule is detected by methods well known in the art (step 5).

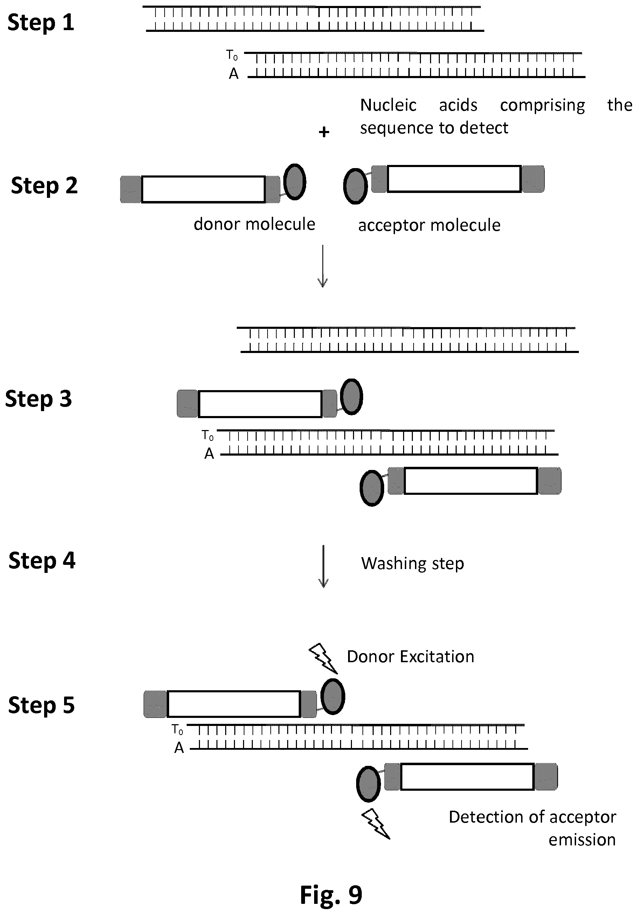

**Figure 9****:** A schematic example of multiplex method. Among nucleic acids (step 1), a nucleic acid sequence of interest is detected by providing two specific DNA binding domains fused respectively to a donor and an acceptor molecule (step2). The DNA binding domains specifically bind different part of the nucleic acid sequence of interest. After contacting the DNA binding domains with nucleic acids (step 3) and removing the unbound DNA binding domains and nucleic acids (step 4), the DNA binding domain: nucleic acid complexes are detected using FRET system: once DNA binding domains are bound on their respective adjacent nucleic acid target sequences, the donor molecule is excited and the increased intensity of the acceptor emission is detected.

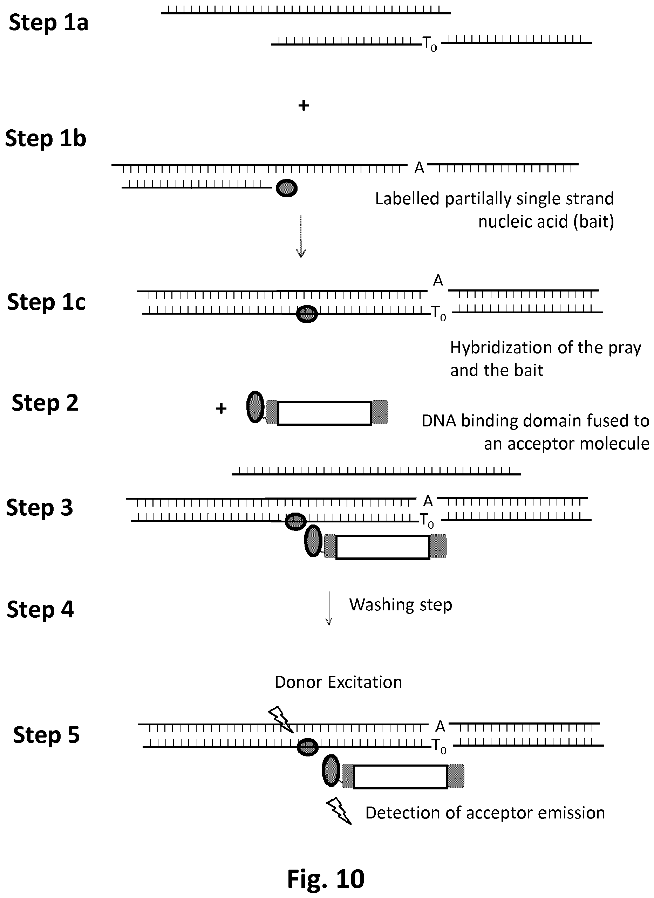

**Figure 10****:** A schematic example of single strand pray detection method using FRET system. To detect the single strand pray sequence among nucleic acids, a labeled partially single strand nucleic acid (bait) comprising a complementary sequence to pray sequence is provided (step 1b). Only the specific pray sequence among nucleic acids hybridizes to the bait sequence under suitable conditions (step 1c). Then, to detect pray:bait sequence, a specific DNA binding domain fused to an acceptor molecule is provided (step 2). After contacting the DNA binding domain with nucleic acids (step 3) and removing the unbound DNA binding domains and nucleic acids (step 4), the labeled nucleic acid is detected using FRET system: the donor molecule excitation induces the transfer of energy to the acceptor, and the increased intensity of the acceptor emission is detected (step 5).

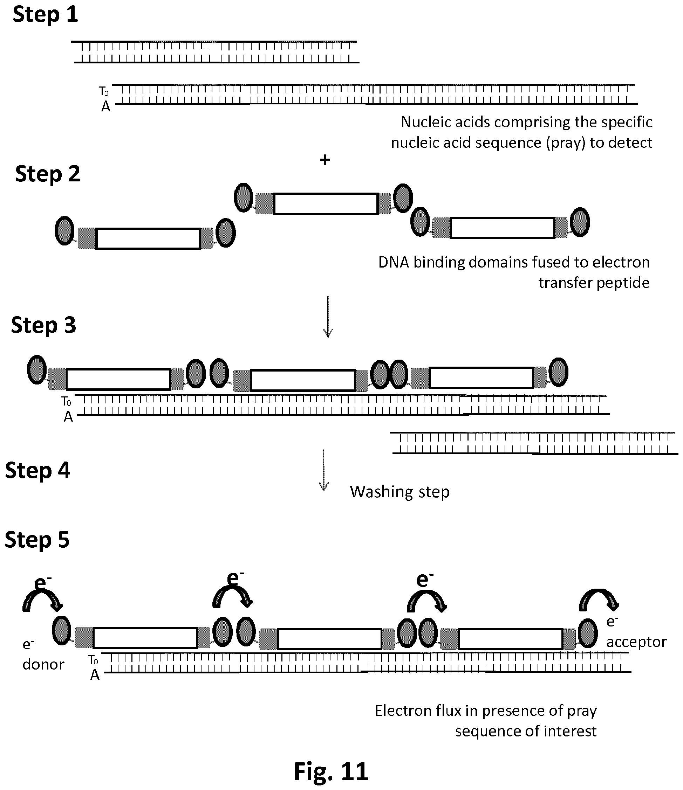

**Figure 11****:** A schematic example of a multiplex detection method using electron flux detection. Among nucleic acids (step 1), a nucleic acid sequence of interest is detected by providing several specific DNA binding domains fused to electron transfer peptides (step2). After contacting the DNA binding domains with nucleic acids (step 3) and removing the unbound DNA binding domains and nucleic acids (step 4), the binding of TALE-detectors on their respective adjacent nucleic acid target sequences, allow the transfer of electrons along the nucleic acid sequence of interest (pray) detected using electron flux detection.

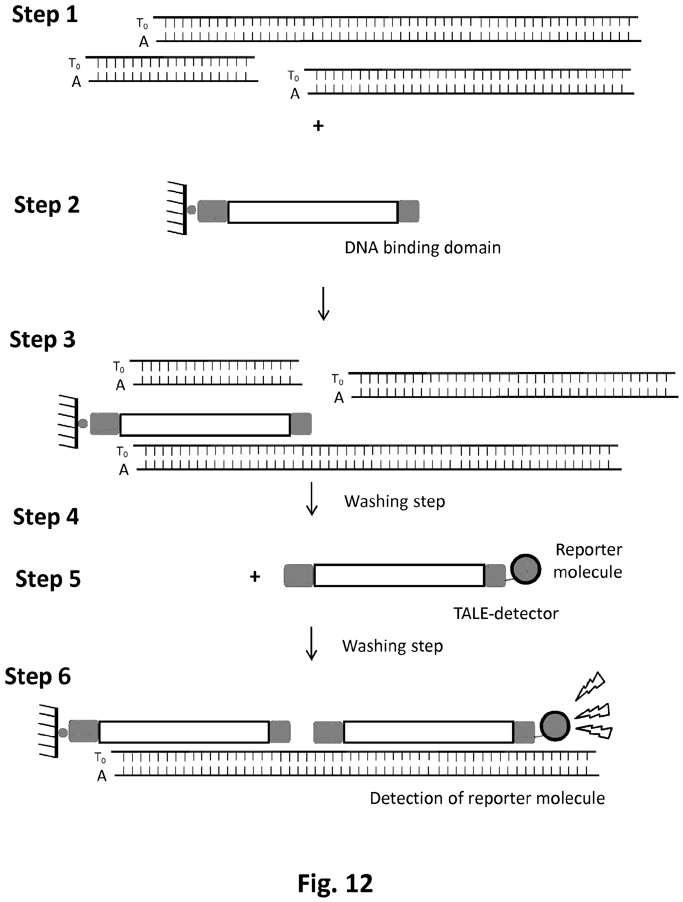

**Figure 12****:** A schematic example of a rapid nucleic acid detection test. Among nucleic acids (step 1), a nucleic acid sequence of interest is detected by providing a first DNA binding domain capable of binding a part of the nucleic acid sequence of interest. The DNA binding domain is immobilized on a solid support (step 2) to capture the nucleic acid sequence of interest (step 3). A second DNA binding domain fused to a reporter molecule is provided. The second DNA binding domain is capable of binding another part of the nucleic acid sequence of interest. After removing the unbound DNA binding domains and nucleic acids (step 4), the reporter molecule is detected by methods well known in the art (step 5).

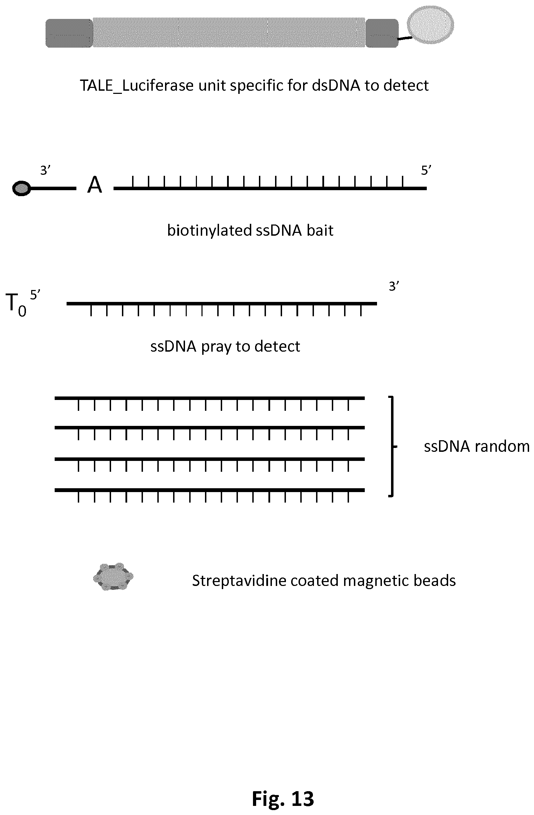

**Figure 13****:** Tale-based diagnostic tool using TALE-Luciferase fusion protein and luminescence as detection read out. The detection method consists in using a TALE DNA binding domain fused to a Renila Luciferase to detect a single strand DNA (ssDNA) sequence of interest via luminescence emission. It relies on strepavidin coated magnetic beads, a biotinylated ssDNA "bait", complementary to the sequence "pray" to detect and a TALE-Luciferase detector protein (TALE-LUC) specific for the double strand DNA (dsDNA) pray:bait complex.

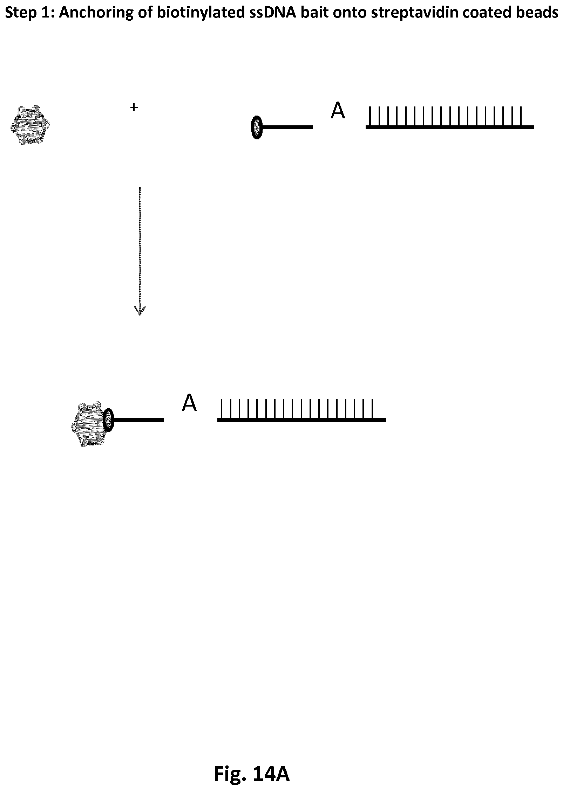

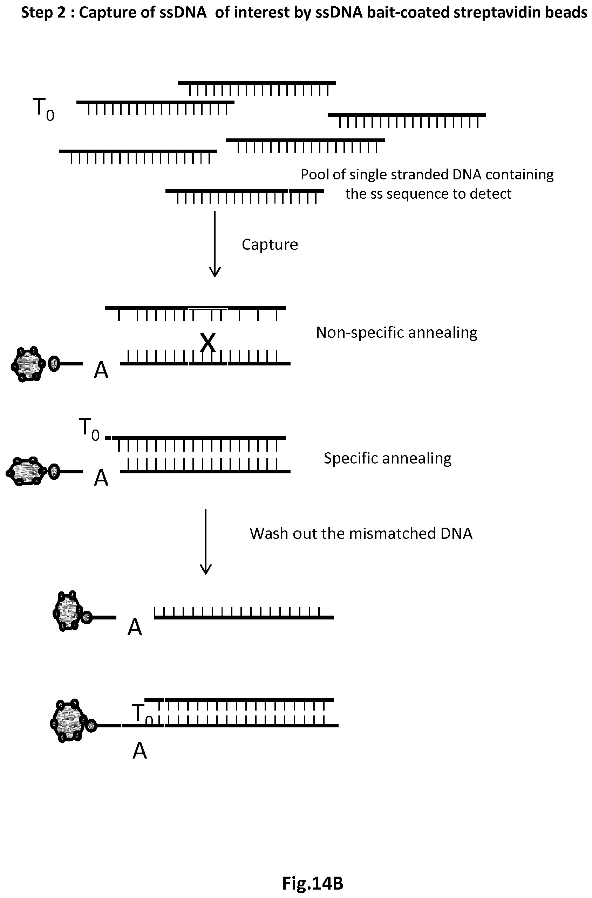

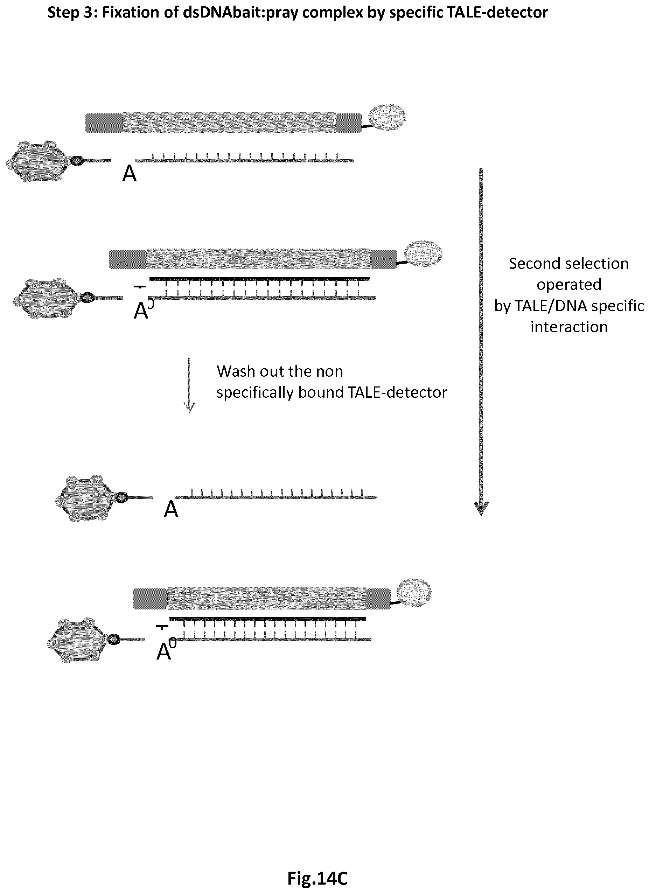

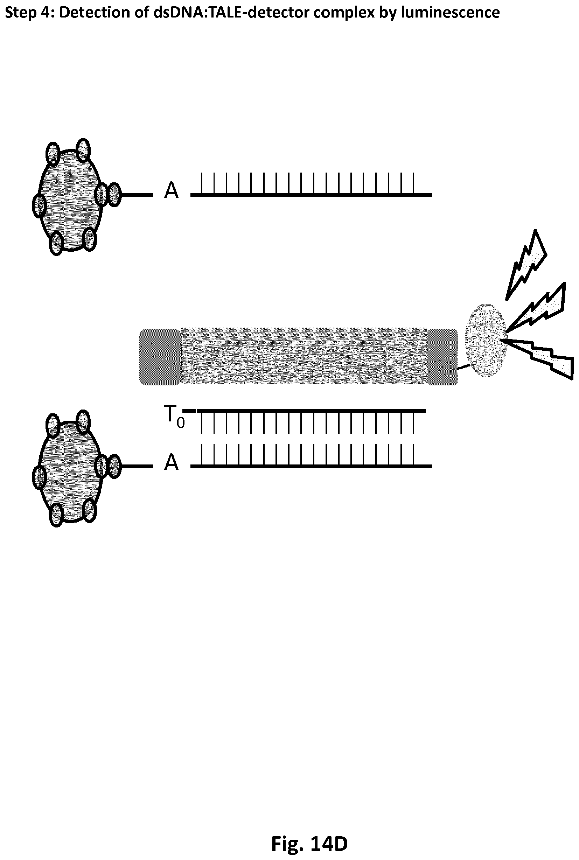

**Figure 14****:** A schematic representation of the different steps involved in the detection of a ssDNA pray. The first step of this method consists in anchoring the ssDNA bait onto the streptavidin coated magnetic beads (Fig.14A, step1). This anchoring step is followed by multiple washing steps to remove unattached ssDNA bait. The pool of ssDNA containing the ssDNA pray to detect is then added to magnetic beads (Fig.14B, step2). In optimized temperature, buffer and salt conditions, the ssDNA pray is expect to anneal with the ssDNA bait in a highly specific manner. This annealing step enables an efficient and specific capture of the DNA pray among the pool of ssDNA molecules. This first capture is then followed by washing steps to remove non specific ssDNA molecules (mismatched DNA). The TALE-LUC, specifically designed to bind the DNA pray strand of the dsDNA pray:bait complex, is then added to the beads and bind to its target specifically (Fig.14C, step 3). This highly specific binding step represents the second capture of the dsDNA to detect. Non specific complex are then washed out and the remaining specific TALE_LUC:dsDNApray:bait complex is eventually detected after addition of luciferase-specific revelation solution and luminometry analysis (Fig.14D, step 4).

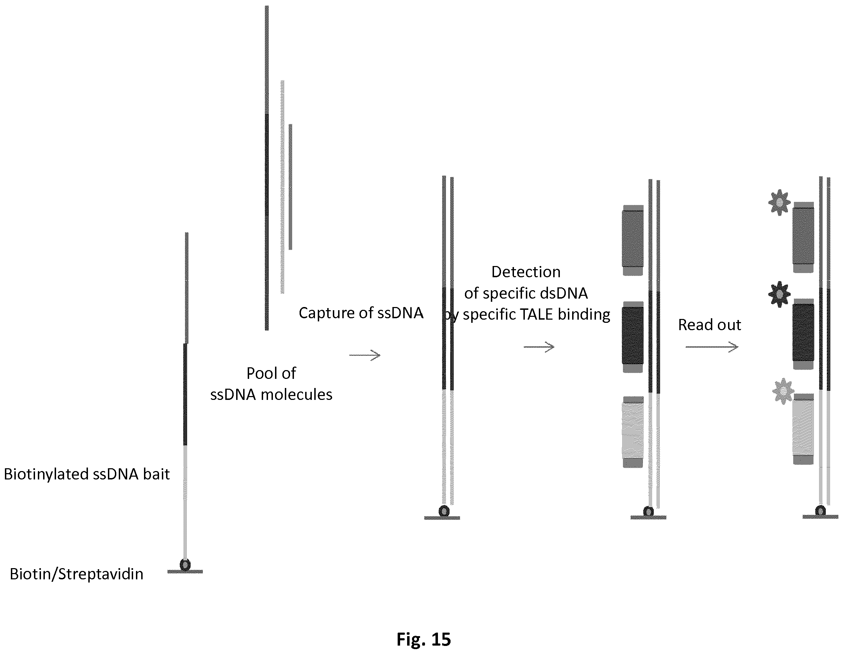

**Figure 15****:** Multiplex detection by using multiple TALE detector protein specifically engineered to emit different signals

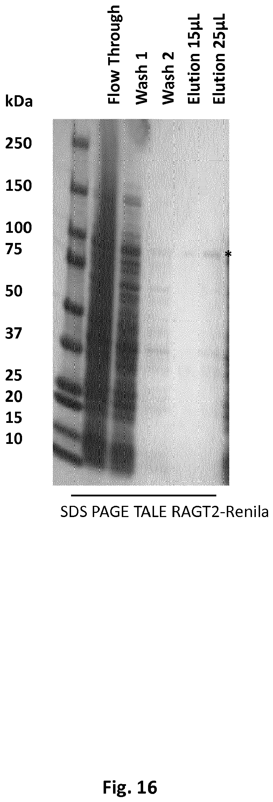

**Figure 16****:** SDS PAGE analysis of TALE RAGT2-Renila detector purification via Nickel affinity column. The different purification steps, including the flow through, the different washing steps and the elution were analyzed. TALE RAGT2-Renila detector is expected at a Mw around 110 kDa. The SDS PAGE band corresponding to TALE-RAGT2-Renila is indicated by a black star.

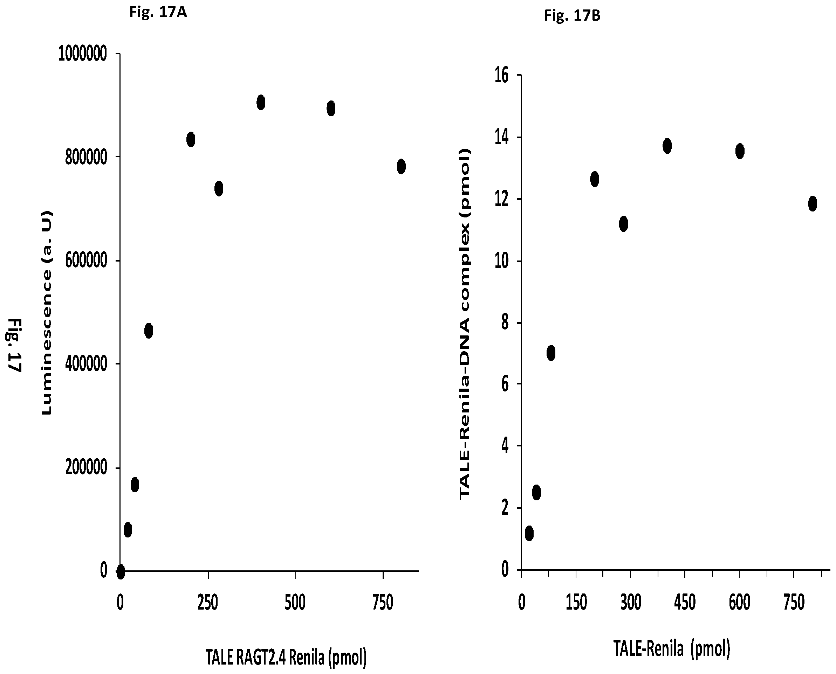

**Figure 17****:** Linearity of Luminescence signal as a function of TALE RAGT2-Renila and sensitivity of TALE RAGT2-RENILA. Each data point is a result of two independent experiments. The non linear portion is indicated in dashed line.

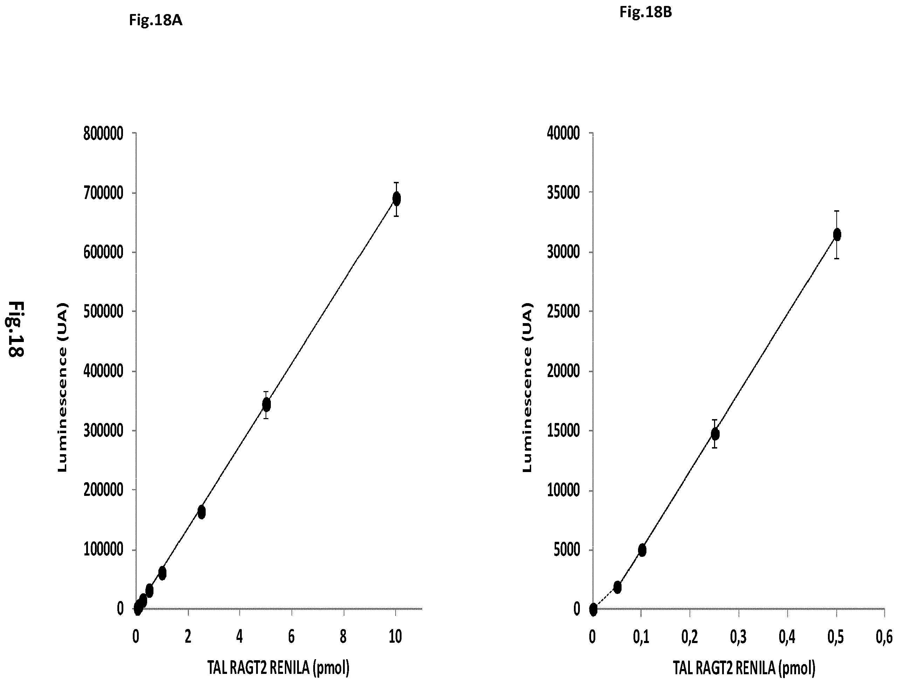

**Figure 18****:** Assessment of the percentage of TALE RAGT2-Renila molecules present in the protein preparation that are able to proficiently bind biotinylated dsDNA (resulting in the annealing of SEQ ID NO 2 and 3).

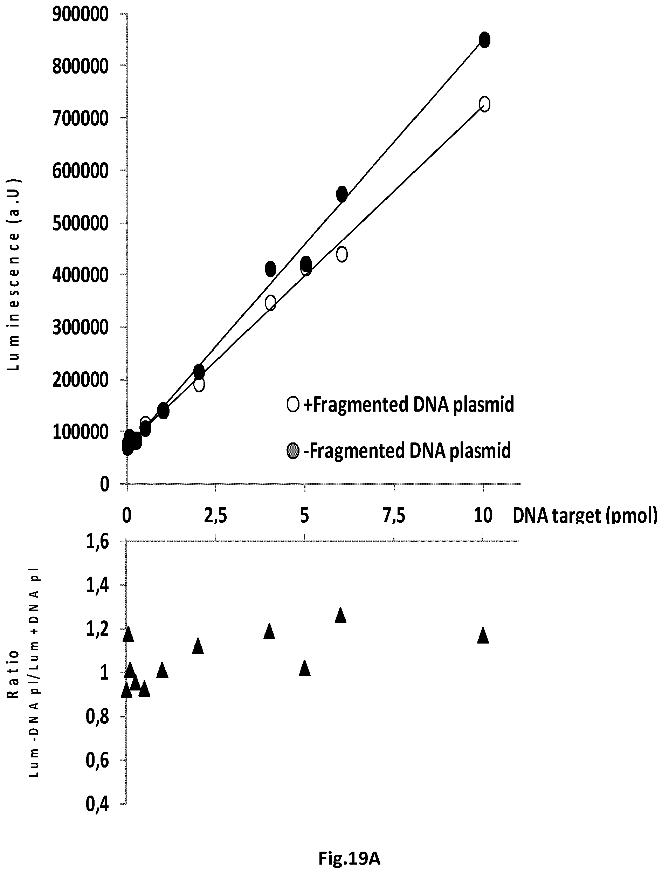

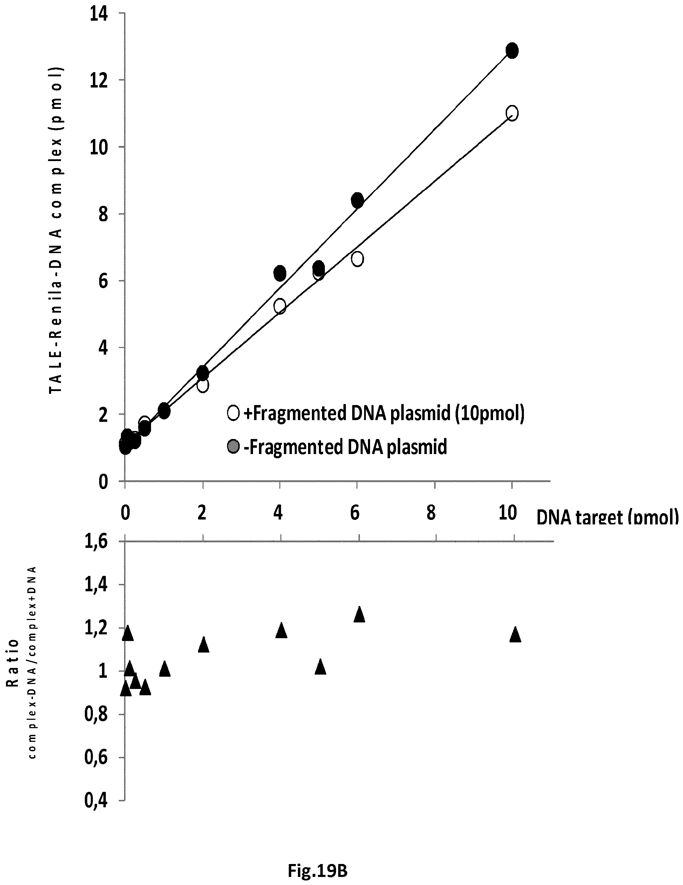

**Figure 19****:** ssDNA pray (SEQ ID NO 5) detection sensitivity of TALE RAGT2-Renila detector and biotinylated ssDNA bait (SEQ ID NO 4) system (Fig.19A: 0.5pmol of ssDNA; Fig.19B: 10pmol of ssDNA).

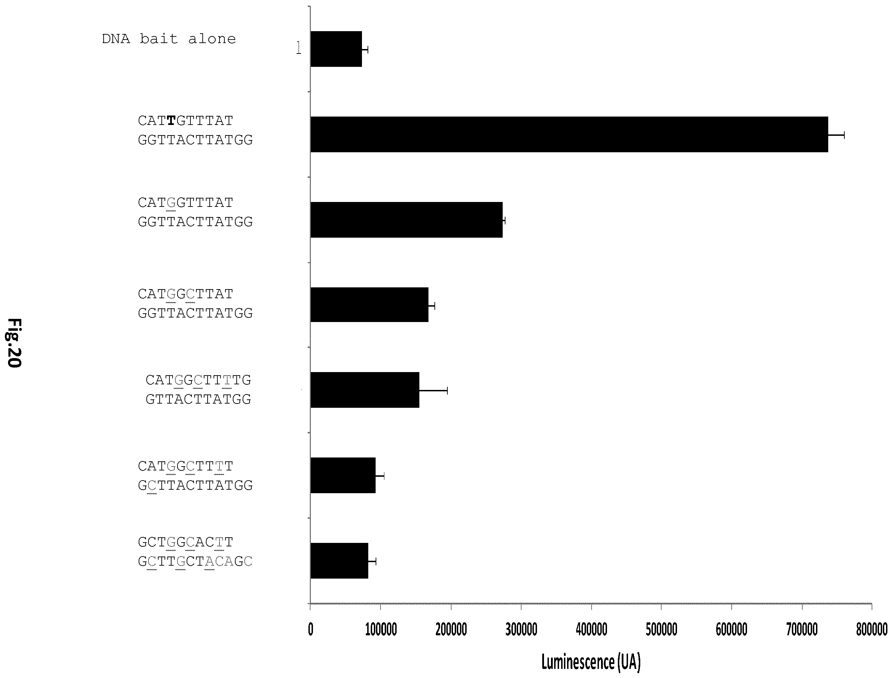

**Figure 20****:** TALERAGT2-Renila detector system specificity assessment. Different ssDNA targets (SEQ ID NO 6-10) harboring unique or multiple mutations were used as ssDNA pray to assess the specificity of TALE RAGT2-Renila detector system. ssDNA pray sequences are indicated on the left side of the figure, oriented from 5'to 3', the T0 position is indicated in black and bold and the different mutations studied are indicated in red. The DNA bait alone (SEQ ID NO 4) was used as negative control. Each data point is a result of two independent experiments.

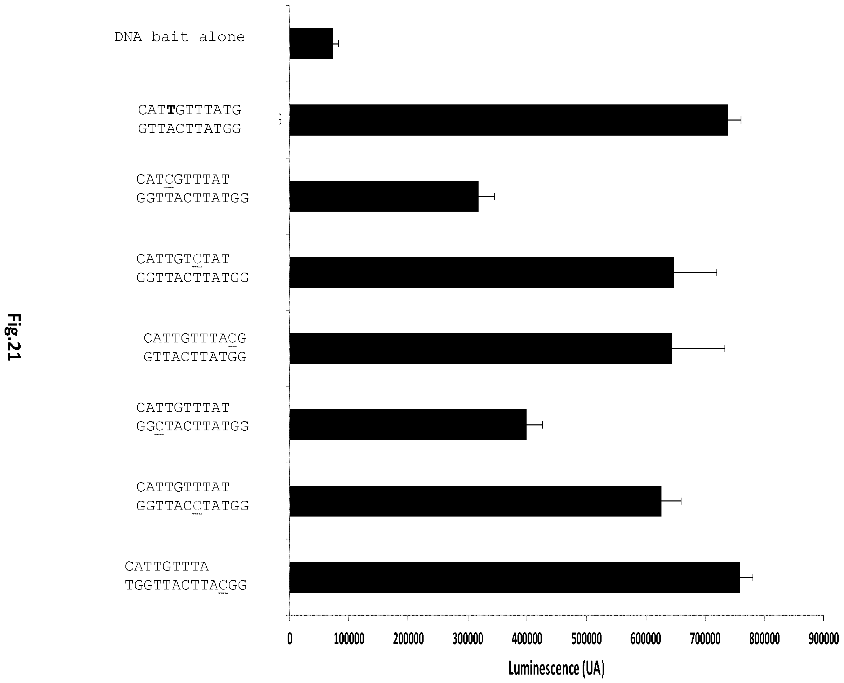

**Figure 21****:** TALE RAGT2-Renila detector system specificity assessments. Positional effect of T to C mutation in the ssDNA (SEQ ID NO 11-16) on the luminescence signal of TALE RAGT2-Renila detector system. ssDNA pray sequences, oriented from 5'to 3', are indicated on the left side of the figure, the T0 position is indicated in black and bold and the different mutations studied are indicated in red. The DNA bait alone (SEQ ID NO 4) was used as negative control. Each data point is a result of two independent experiments.

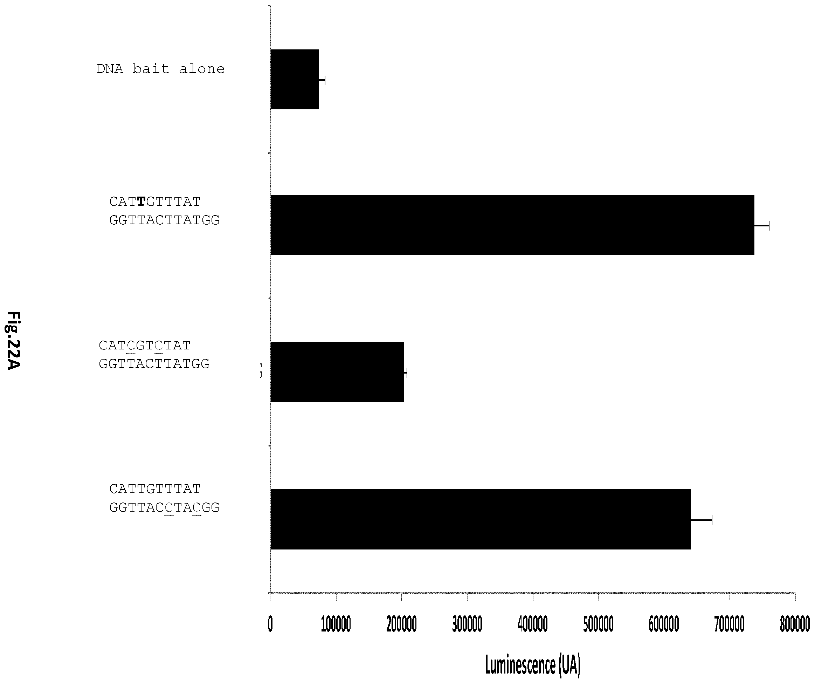

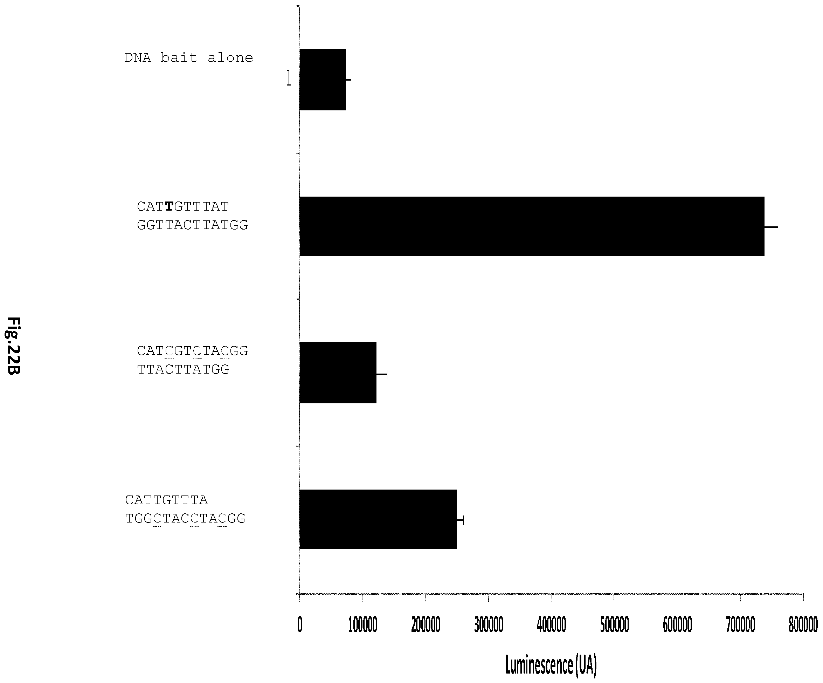

**Figure 22****:** TALE RAGT2-Renila detector system specificity assessments. Positional effect of two or three T to C mutations in the ssDNA pray on the luminescence signal of TALE RAGT2-Renila detector system. Fig.22A and Fig.22B show mutations at the 5' end and 3'end of the ssDNA pray respectively. ssDNA pray sequences (SEQ ID NO 17-20), oriented from 5'to 3', are indicated on the left side of the figure, the T0 position is indicated in black and bold and the different mutations studied are indicated in red. The DNA bait alone (SEQ ID NO 4) was used as negative control. Each data point is a result of two independent experiments.

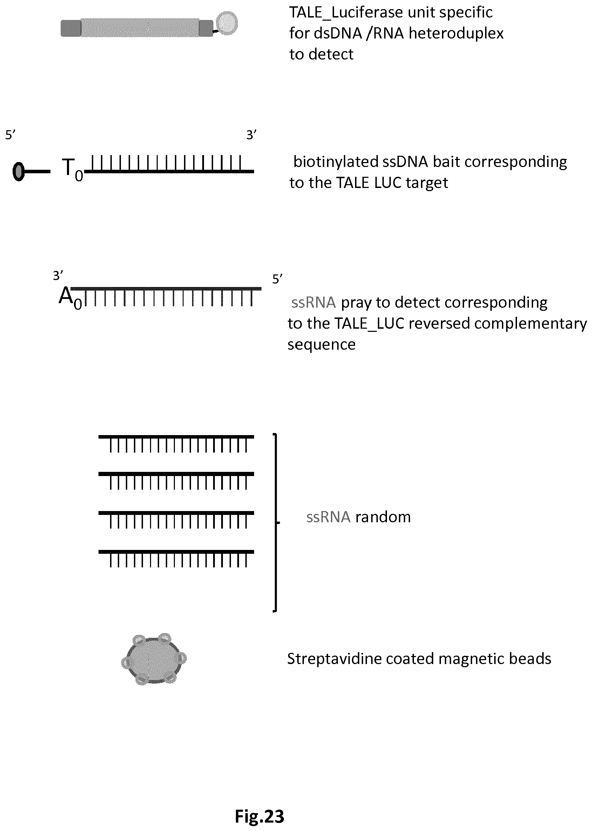

**Figure 23****:** Schematic representation of the different elements used for the detection of a ssRNA pray.

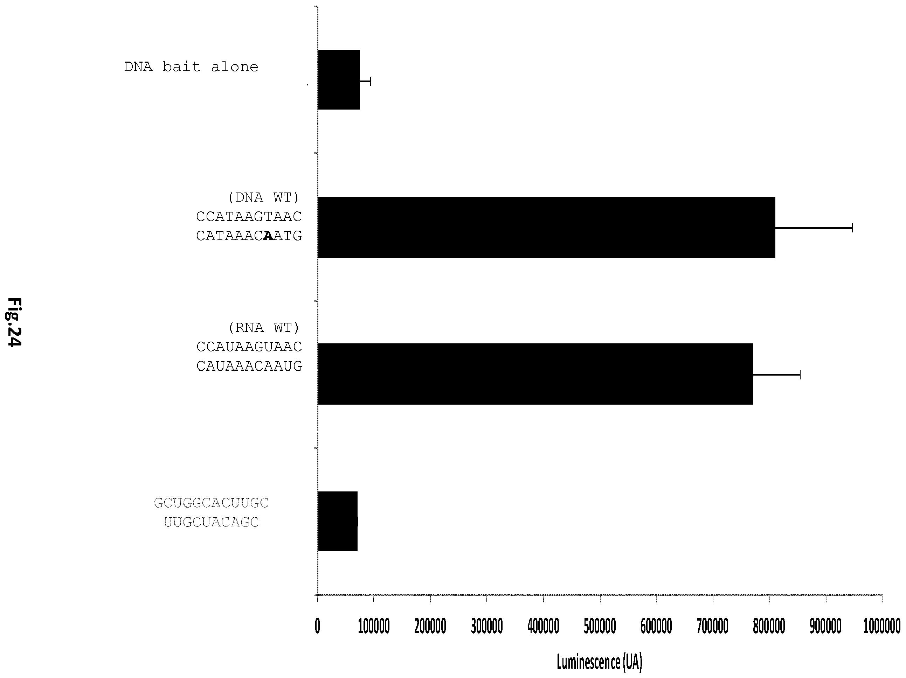

**Figure 24****:** Assessment TALE RAGT2-Renila detector system capacity to detect ssRNA molecules. ssDNA and ss DNA pray sequences (SEQ ID NO 22-23), oriented from 5'to 3', are indicated on the left side of the figure, the A0 position (complementary to the T0 sense strand position) is indicated in black and bold and the random ssRNA pray sequence is indicated in red. Each data point is a result of two independent experiments. Three control experiments were performed with ssDNA pray (SEQ ID NO 22, positive control), with random ssRNA pray and with the wild type ssDNA bait alone (SEQ ID NO 23 and 21 respectively).

### Description of the invention

The present invention relates to a new nucleic acid diagnostic tool based on TALE-protein. In particular, the present invention relates to a method of detecting a nucleic acid sequence of interest as described in the claims.

### Nucleic acid sequence of interest

The nucleic acid sequence of interest can be a genetic sequence present within a cell, such as, for example a gene or a non-coding sequence within or adjacent to a gene. The nucleic acid sequence of interest can be present in a chromosome, an episome, an organellar genome such as mitochondrial or chloroplast genome or genetic material that can exist independently to the main body of genetic material such as an infecting viral genome, plasmids, episomes, transposons for example. A sequence of interest can be within the coding sequence of a gene, within transcribed non-coding sequence such as, for example, leader sequences, trailer sequence or introns. As TALE DNA binding domains have the ability to bind DNA-RNA heteroduplex with a similar affinity than double strand DNA, in the present invention nucleic acid can be DNA, RNA or DNA-RNA heteroduplex.

### Single strand nucleic acid

The nucleic acid sequence of interest is a single strand nucleic acid sequence. In particular, to detect a single strand nucleic acid sequence of interest (pray sequence), a nucleic acid sequence (bait sequence) complementary to at least to a part of the sequence of interest is provided. Among nucleic acids, only the specific pray sequence hybridized to the bait sequence. To detect the pray:bait sequence, at least one specific DNA binding domain capable of binding said hybridized sequence is provided. As described above, after contacting said DNA binding domain with nucleic acids and removing unbound DNA binding domain and nucleic acids, the presence or absence of the nucleic acid sequence of interest is detected. As TALE DNA binding domains display a much lower affinity for single stranded nucleic acid than for their double stranded counterparts, the low affinity binding of TALE domain with single strand DNA (ssDNA) avoids the formation of TALE:ssDNA complex rendering this method particularly sensitive.

In other words, the method of detecting a single-strand nucleic acid sequence of interest, named "pray" comprises:
(a) Providing nucleic acids which can comprise a single strand nucleic acid sequence of interest (pray sequence);
(b) Providing a nucleic acid comprising a single strand sequence complementary to a part of said pray sequence (bait);
(c) Hybridizing said pray to said bait;
(d) Providing at least one DNA-binding domain capable of binding said hybridized pray:bait sequence;
(e) Contacting DNA binding domain with hybridized pray:bait sequence;
(f) Removing unbound DNA binding domain and nucleic acids
(g) Detecting the presence or the absence of the pray.
Examples of this method are represented in Figure 2, 5, 7 and 10.

According to the invention, the nucleic acid sequence named bait comprises a single-strand complementary sequence of at least a part of the pray such that the two single-strand oligonucleotides can hybridize together. By hybridization sequence is meant the sequence part of the oligonucleotide that can hybridize to one of the other oligonucleotides under standard low stringent conditions. Such conditions can be for instance at room temperature for 2 hours by using a buffer containing 25% formamide, 4x SSC, 50 mM NaH2PO4 / Na2HPO4 buffer; pH 7.0,5x Denhardt's, 1 mM EDTA,1 mg/ml DNA + 20 to 200 ng/ml probe to be tested (approx. 20 - 200 ng/ml). This can be also predicted by standard calculation of hybridization using the number of complementary bases within the sequence and the content in G-C at room temperature as provided in the literature.

Preferentially, the hybridization sequences are complementary to each other pursuant to the complementarity between two nucleic acid strands relying on Watson-Crick base pairing between the strands, i.e. the inherent base pairing between adenine and thymine (A-T) nucleotides and guanine and cytosine (G-C) nucleotides. Accurate base pairing equates with Watson-Crick base pairing includes base pairing between standard and modified nucleosides and base pairing between modified nucleosides, where the modified nucleosides are capable of substituting for the appropriate standard nucleosides according to the Watson-Crick pairing. The complementary sequence of the single-strand oligonucleotide can be any length that supports specific and stable hybridization between the two single-strand oligonucleotides under the reaction conditions.

In one embodiment, said bait can be a single strand nucleic acid sequence which comprises a complementary sequence to a part of the pray sequence. In another embodiment, said bait can be a nucleic acid sequence comprising a double-stranded sequence and a single strand sequence which comprises a complementary sequence to a part of the pray sequence (see Figure 10).

### Sample:

The nucleic acid sequence of interest in the present invention can be detected *in situ* within a live cell. The DNA-binding domain, preferably polynucleotides encoding said DNA binding domain can be introduced into the live cells by transformation methods well-known in the art including as non limiting examples electroporation, microinjection, sonoporation, particle bombardment, liposome transfection and delivery via viral vector. The specific nucleic acid sequence in the present method can be also detected *in situ* within a cell or tissue, preferably fixed on microscope slides.

In particular embodiment, the nucleic acid sequence of interest of the present invention can be present in a sample. The sample of the invention is any type of sample which may contain the desired nucleic acid species, such as e.g. a reaction mixture. It may also be, for example, a biological sample. A "biological sample" may be any sample which may contain a biological organism, such as, for example, bacteria, viruses, plants, yeasts etc. A "biological sample" according to the invention also refers to a sample which may be obtained from a biological organism, such as a cellular extract obtained from bacteria, viruses, plants, yeasts etc. in particular, a biological sample may be any sample taken from a subject, such as a serum sample, a plasma sample, a urine sample, a blood sample, a lymph sample, or a biopsy. Preferred biological samples for the detection of a specific nucleic acid sequence among genomic sequences include samples such as a blood sample, a plasma sample, a lymph sample, or a biopsy. Preferably, the biological sample is a blood sample. A blood sample can be used from a cancer patient and, for example, used to characterize a liquid tumor by the method of the invention. A "biological sample" as used herein also includes a solid cancer sample of the patient to be tested, when the cancer is a solid cancer.

The sample may thus be any material containing nucleic acid within such cells, including for example foods and allied products, clinical and environmental samples. Thus, the sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. Representative samples thus include whole blood and blood-derived products such as plasma or buffy coat, urine, faeces, cerebrospinal fluid or any other body fluids, tissues, cell cultures, cell suspensions etc., and also environmental samples such as soil, water, or food samples. The sample may also include relatively pure or partially purified starting materials, such as semi-pure preparations obtained by other cell separation process

### DNA binding domain

By "DNA binding domain", it is meant a protein domain capable of binding a nucleic acid sequence of interest, preferably a double strand nucleic acid molecule. The DNA binding domain recognizes and binds nucleic acid at specific polynucleotide sequences, further referred to as "nucleic acid target sequence". In the invention, said DNA binding domain is derived a Transcription Activator like Effector (TALE) engineered to bind a specific nucleic acid target sequence.

TALEs are proteins from the bacterial species *Xanthomonas* which comprise a plurality of repeat sequences, each repeat comprising di-residues in position 12 and 13 (RVD) that are specific to each nucleotide base of the nucleic acid targeted sequence. TALE binding domain is composed by a variable number of 33-35 amino acid repeat modules. These repeat modules are nearly identical to each other except for two variable amino acids located at positions 12 and 13 (i.e. Repeat Variable Di residues, RVD). The nature of residues 12 and 13 determines base preferences of individual repeat module. Preferably, RVDs associated with recognition of the different nucleotides are HD for recognizing C, NG for recognizing T, NI for recognizing A, NN for recognizing G or A, NS for recognizing A, C, G or T, HG for recognizing T, IG for recognizing T, NK for recognizing G, HA for recognizing C, ND for recognizing C, HI for recognizing C, HN for recognizing G, NA for recognizing G, SN for recognizing G or A and YG for recognizing T, TL for recognizing A, VT for recognizing A or G and SW for recognizing A. In another embodiment, critical amino acids 12 and 13 can be mutated towards other amino acid residues in order to modulate their specificity towards nucleotides A, T, C and G and in particular to enhance this specificity.

Binding domains with similar base-per-base nucleic acid binding properties (modular base-per-base specific nucleic acid binding domains (MBBBD) derived from new modular proteins recently discovered by the applicant in a different bacterial species (WO2014/018601) are also known. Said MBBBD can be engineered, for instance, from the newly identified proteins, namely EAV36_BURRH, E5AW43_BURRH, E5AW45_BURRH and E5AW46_BURRH proteins from the recently sequenced genome of the endosymbiont fungi *Burkholderia Rhizoxinica* (Lackner, Moebius et al. 2011; Lackner, Moebius et al. 2011).

In another embodiment, the method according to the present invention relates to a multiplex detection method (Figure 3). The multiplex detection method comprises in step (b) or (d) as described above, providing several DNA binding domains each one capable of binding a part of said nucleic acid sequence of interest. Different multiplex methods are represented in Figures 8, 9, 11 and 12. This method can comprise providing one or more DNA binding domain(s), preferably one, two, three, four or more DNA binding domains capable of binding said nucleic acid sequence of interest.

### Solid support

In a particular embodiment, the bound of the nucleic acid sequence of interest with the DNA binding domain (optionally via the bait) can be realized in solution or on a solid support.

In a particular embodiment, said DNA-binding domain and/or said nucleic acid sequence is fixed on a solid support (see for examples Figures 4, 5 and 12). The solid support may be any of the well known supports or matrices which are currently widely used or proposed for immobilization, separation etc. These may take the form of particles, beads, sheets, gels, filters, membranes, fibres, capillaries, or microtitre strips, tubes, plates or wells etc. Conveniently the support may be made of glass, silica, latex or a polymeric material. Such supports will generally have an irregular surface and may be for example be porous or particulate eg. particles, fibres, webs, sinters or sieves. Particulate materials eg. beads are generally preferred due to their greater binding capacity, particularly polymeric beads. However, to aid manipulation and separation, magnetic beads are preferred. Thus, in the method of the invention, the magnetic particles with cells attached may be removed onto a suitable surface by application of a magnetic field eg. using a permanent magnet. It is usually sufficient to apply a magnet to the side of the vessel containing the sample mixture to aggregate the particles to the wall of the vessel and to pour away the remainder of the sample.

Immobilisation may be achieved in any number of ways, known in the art, described herein, and/or as can be developed. For example, immobilisation may involve any technique resulting in direct and/or indirect association of an analyte (and its corresponding antagonist) with the substrate, including any means that at least temporarily prevents or hinders its release into a surrounding solution or other medium. The means can be by covalent bonding, non-covalent bonding, ionic bonding, electrostatic interactions, Hydrogen bonding, van der Waals forces, hydrophobic bonding, or a combination thereof. For example, immobilisation can be mediated by chemical reaction where the substrate contains an active chemical group that forms a covalent bond with the second binding partner. For example, an aldehyde-modified support surface can react with amino groups in protein receptors; or amino-based support surface can react with oxidization-activated carbohydrate moieties in glycoprotein receptors; a support surface containing hydroxyl groups can react with bifunctional chemical reagents, such as N,N dissuccinimidyl carbonate (DSC), or N-hydroxysuccinimidyl chloroformate, to activate the hydroxyl groups and react with amino-containing receptors. In some embodiments, support surface of the substrate may comprise animated or carboxylated polystyrenes; polyacrlyamides; polyamines; polyvinylchlorides, and the like. In still some embodiments, immobilization may utilize one or more binding-pairs to bind or otherwise attach a receptor to a substrate, including, but not limited to, an antigen-antibody binding pair, hapten/anti-hapten systems, a avidin-biotin binding pair; a streptavidin-biotin binding pair, a folic acid/folate binding pair; photoactivated coupling molecules, and/or double stranded oligonucleotides that selectively bind to proteins, e.g., transcriptional factors.

### Detection methods

By "detecting a nucleic acid sequence of interest", it is herein meant all the activities leading directly or indirectly to the obtaining of some information on the presence or absence of a nucleic acid sequence of interest, or eventually the quantity of a nucleic acid of interest including but not limited to, the detection and/or quantification of the DNA binding domain, the detection and /or quantification of at least a part of the nucleic acid molecule and/or the detection and/or the quantification of the DNA binding domain bound to the specific nucleic acid molecule. The detection/and or quantification of a specific nucleic acid sequence can be realized by techniques that allow the specific separation of the nucleic acid sequence bound to DNA-binding domain from nucleic acid which do not have bound DNA binding domain thereon.

The detection and/or quantification of the specific nucleic acid sequence can be realized by techniques well known in the art that allow the detection and/or quantification of the DNA binding domain included as non limiting examples, immunoassay, protein immunoprecipitation, mass spectrometry, colorimetry methods (e.g Biuret assay, Lowry assay, bis-cinchinonic acid assay, Bradford assay), amino acid analysis, and/or the detection and/or quantification of the specific nucleic acid sequence included as non limiting examples PCR, real time PCR, sequencing, electrophoresis.

The detection and/or quantification of a specific nucleic acid sequence may include the detection of a reporter molecule fused to the DNA binding domain and labeled bait nucleic acid of the present invention (as illustrated in Figure 6 and 7). Said reporter molecule can be fused to the N-terminal and/or C-terminal end of the DNA binding domain. A reporter gene is one whose transcription is detectable and/or which expresses a protein which is also detectable, either of which can be assayed. Examples of readily detectable proteins include β-galactosidase, fluorescent protein (e.g. green fluorescent protein (GFP), red, cyan, yellow fluorescent proteins), chemiluminescent protein, a radioisotope, a tag marker (e.g. HA, FLAG), luciferase, beta-galactosidase, beta lactamase, alkaline phosphatase and chloramphenicol acetyl transferase as well as enzymes or proteins, i.e. selectable markers, involved in nutrient biosynthesis such as Leu2, His3, Trp1, Lys2, Ade2 and Ura3.

Said nucleic acid can be labeled with several fluorescent protein or enzymes that generate colored products. The nucleic acid used in the present invention can be labeled with more than one distinguishable fluorescent or pigment color. Nucleic acids may be labeled with Texas red, rhodamine and its derivatives, fluorescein and its derivatives, dansyl, umbelliferone and the like or with horse radish peroxidase, alkaline phosphatase, or the like.

Nucleic acid can be labeled directly or indirectly. The common indirect labeling schemes covalently bind a ligand to the nucleotide and prepare labeled nucleic acid by incorporating this using random priming or nick translation. The ligand then binds an anti-ligand which is covalently bound to a fluorescent label. Ligands and anti- ligands vary widely. When a ligand has an anti-ligand, e.g., biotin, thyroxine, or cortisol, the ligand may be used in conjunction with the labeled naturally-occurring anti-ligand. Alternatively, a hapten or antigen may be used in combination with an antibody.

In another embodiment, the detection and/or quantification of the specific nucleic acid sequence can also be based on protein complementation assays wherein a reporter molecule is dissected into two non-functional fragments. Functionality is restored when the fragments are reassembled by attached protein-protein interaction domains. Reporter molecules used in the protein complementation assays can be a β-galactosidase, dihydrofolate reductase, fluorescent protein (e.g. green fluorescent protein, GFP), luciferase, β-lactamase. Detection of the signal from reporter molecule may be done by standard methods known in the art for diagnostic and other detection methods such as fluorescence or colorimetric detection systems. One example of methods according to the present invention using protein complementation assay is illustrated in Figure 8.

FRET (Fluorescence resonance energy transfer) occurs when the emission spectrum of a fluorescent molecule, an energy donor, overlaps with the absorption spectrum of another molecule, an energy acceptor. When the donor is excited, FRET results in reduction of the intensity of donor emission, as energy from the donor in its excited state is transferred to the acceptor. The acceptor can be fluorescent or non- fluorescent. If the acceptor is fluorescent, the intensity of acceptor emission is increased as a result of FRET. A second critical criterion of energy transfer in FRET is close proximity between energy donor and acceptor. In a particular embodiment, the acceptor can be fused to the DNA binding domain and the acceptor can be fused to the nucleic acid or inversely the acceptor can be fused to the nucleic acid and the donor can be fused to the nucleic acid such that when the DNA binding domain specifically binds target nucleic acid the intensity of the acceptor emission is increased. Examples of methods using FRET techniques are illustrated in Figures 9 and 10.

The present invention may also use a DNA binding domain fused to at least one electron transfer peptide. In a more particularly embodiment, the present invention may use a nanowire which comprises several DNA binding domains fused to an electron transfer peptide which are adjacent on nucleic acid target sequence, such that each electron transporter are regularly disposed along the nucleic acid molecule to allow the transfer of electrons. In a more preferred embodiment said electron transfer peptide is derived from an Iron-Sulfur protein (Fe-S protein). By "a peptide derived from", it is meant a peptide having an amino acid sequence which comprises at least a part of amino acid sequence of a Fe-S protein, or a variant thereof. Iron-sulfur proteins are proteins characterized by the presence of iron-sulfur clusters containing sulfide-linked di-, tri-, and tetrairon centers in variable oxidation states (WO2012/120013). As non limiting examples, Fe-S protein can be rubredoxin (i. e. NCBI Reference Sequence: WP_013180622.1), ferredoxin, hydrogenase or variants thereof. Also disclosed herein is a method of producing said DNA binding domain fused to an electron transfer peptide comprising introducing into a cell at least one polynucleotide encoding said DNA binding domain fusion protein, and expressing DNA binding domain fusion protein into the cell. Also disclosed herein is a polynucleotide, an expression vector encoding said DNA binding domain fused to an electron transfer peptide, or a host cell comprising said polynucleotide or DNA binding domain fusion protein.

In the context of the reporter molecule, the DNA binding domain fused to an electron transfer peptide can be used to detect the presence or absence of the DNA binding domain, for example by detecting the production of electrons. One example of methods using TALE DNA binding domain fused to an electron transfer peptide is represented in Figure 11.

"Detecting nucleic acid sequence" can also refer to the quantification of the specific nucleic acid sequence. The quantity of nucleic acid sequence and/or DNA binding domain can be determined by comparing the result with standard sample comprising known amounts of a purified protein (e. g. bovine serum albumin (BSA)) or of nucleic acid. In another embodiment, said nucleic acid can be quantified by a competitive binding assay based for example on the competitive nucleic acid binding between labeled DNA binding domain and unlabelled DNA binding domain, or competitive DNA binding domain binding between labeled nucleic acid and unlabeled nucleic acid.

### Applications

The present invention can thus be used every time there is a need to detect nucleic acid sequence of interest. Applications include the fields of in vitro diagnostics, including clinical diagnostics, research in the fields of molecular biology, high throughput drug screening, veterinary diagnostics, agricultural-genetics testing, environmental testing, food testing, industrial process monitoring, biosecurity, forensics, and insurance testing. In vitro diagnostics and clinical diagnostics is related to the analysis of nucleic acid samples drawn from the body to detect the existence of a disease or condition, its stage of development and/or severity, and the patient's response to treatment. In high throughput drug screening and development nucleic acids are used similarly to other agents, such as, antigens, antibodies, receptors, etc., to analyze the response of biological systems upon exposure to libraries of compounds in a high sample number setting to identify drug leads. Veterinary diagnostics and agricultural genetics testing involve samples from a non-human animal or a plant species similar to in vitro diagnostics and to provide means of quality control for agricultural genetic products and processes. In environmental testing, organisms and their toxins that indicate the pollution of an environmental medium, e.g. soil, water, air, etc., are analyzed. Food testing includes the quantitation of organisms, e.g. bacteria, fungi, etc., as a means of quality control. In industrial process monitoring, nucleic acids are detected to indicate proper control of a production process and/or to generate a signal if such processes are out of control. In insurance testing organisms and/or their toxins are identified in screening tests to determine the risk category of a client or to help approve candidates. There are various other applications of the detection of nucleic acids and new applications are being developed constantly.

In particular embodiment, the method according to the present invention can be used to detect the presence or absence of a nucleic acid sequence of interest such as a genetic abnormality or a single nucleotide polymorphism (SNP). This system can be used to detect genomic rearrangement in DNA and for identification of highly repetitive sequence. The method can also be used to detect specific nucleic acid, that serve as a unique marker for a disease or type of cancer or to detect the presence or absence of a nucleic acid sequence of an infectious agent such as HIV, influenza, or *E.coli.* The method according to the present invention can also be used for DNA profiling studies.

### Kit

Also herein disclosed is a kit comprising the DNA binding domain as described above, specific to a nucleic acid sequence and preferably fused to a reporter molecule. The kit according to the present disclosure can be used for all above-mentioned applications. The kit is particularly useful to detect the presence or absence of a specific nucleic acid sequence such as a genetic abnormality or a single nucleotide polymorphism (SNP). The kit can also be used to detect genomic rearrangement in DNA, for identification of highly repetitive sequence, to detect specific nucleic acid that serves as a unique marker for a disease or type of cancer, and to detect the presence or absence of nucleic acid sequence of interest of an infectious agent. Additionally, the kit preferably comprises the reagents for extraction of the biological sample to be tested, optionally a resuspension solution and/or a substrate for assaying the presence of the binding event.

### Definitions:

- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.

The term "nucleic acid" refers to deoxyribonucleotide or ribonucleotide polymer, in linear or circular conformation, and in either single- or double-stranded form. The terms can encompass known modified nucleotides.

As used in the context of the present invention, the term "modified nucleotide" refers to a nucleotide that differs in structure from the standard or "unmodified" nucleotides 2 '-deoxy- adenosine, 2'-deoxythymidine, 2'-deoxy-cytidine and 2'-deoxy-guanosine, and that is capable of pairing with an unmodified nucleotide or a modified nucleotide. Non limiting examples of modifications on the sugar or base moieties of a nucleotide include the addition (or removal) of acetyl groups, amino groups, carboxyl groups, carboxymethyl groups, hydroxyl groups, methyl groups, phosphoryl groups and thiol groups, as well as the substitution of the carbon and nitrogen atoms of the bases with other atoms (e.g., 7-deaza purines). Modified nucleotide also include dideoxy nucleotides, 2'-O-methyl nucleotides, locked nucleic acids (LNA), peptides nucleic acids (PNA), and morpholinos. The nucleotides of the single-stranded nucleic acids may be linked by phosphodiester, phosphorodiamate bonds, or combinations thereof. Such modified nucleotides are useful for instance to introduce markers, tag, epitopes or reactive groups into the genome. The linear single-strand oligonucleotide can also comprise one or more phosphorothioatephosphodiester bonds between terminal base pairs to protect the linear donor polynucleotide from exonucleolytic degradation. These bonds may be in two or more positions at the 5' and/or 3' ends of the molecule and may be added during isolation or synthesis using standard methodology. See, e.g. (Ciafre, Rinaldi et al. 1995). Said single-strand oligonucleotide may comprise phosphodiester linkages, phosphorothiorate linkages, phosphoramidite
- By "gene" is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns, a 3' untranslated region. The gene may further comprise a terminator, enhancers and/or silencers.
- By " delivery vector" or " delivery vectors" is intended any delivery vector which can be used to put into cell contact or deliver inside cells or subcellular compartments agents/chemicals and molecules (proteins or nucleic acids) needed in the present invention. It includes, but is not limited to, transducing vectors, liposomal delivery vectors, viral delivery vectors, drug delivery vectors, chemical carriers, polymeric carriers, lipoplexes, polyplexes, dendrimers, microbubbles (ultrasound contrast agents), nanoparticles, emulsions or other appropriate transfer vectors. These delivery vectors allow delivery of molecules, chemicals, macromolecules (genes, proteins), or other vectors such as plasmids. These delivery vectors are molecule carriers.

By "variant(s)", is meant a protein or a polynucleotide encoding thereof that are obtained by genetic engineering or by random mutagenesis. A variant can for example be obtained by deletion, addition or substitution with a different amino acid of at least one residue in the amino acid sequence of their wild-type sequences. Substitution(s), additions(s) and deletion(s) can for example be introduced by directed mutagenesis and/or by random mutagenesis. In the frame aspects of the present disclosure, variants have the same function and ability than the parent protein or polynucleotide.
- By "DNA binding domain", it is meant a protein domain capable of binding a target nucleic acid sequence, preferably a DNA molecule. The DNA binding domain recognizes and binds nucleic acid at specific polynucleotide sequences, further referred to as "nucleic acid target sequence". The DNA binding domain is derived from a Transcription Activator like Effector (TALE).

Also disclosed herein is that binding domains with similar base-per-base nucleic acid binding properties (modular base-per-base specific nucleic acid binding domains (MBBBD) (WO2014/018601) can also be derived from new modular proteins recently discovered by the applicant in a different bacterial species. Said MBBBD can be engineered, for instance, from the newly identified proteins, namely EAV36_BURRH, E5AW43_BURRH, E5AW45_BURRH and E5AW46_BURRH proteins from the recently sequenced genome of the endosymbiont fungi *Burkholderia Rhizoxinica.*

Also disclosed herein is that the binding domain can also be derived from the CRISPR/Cas9 system. Recently, a new genome engineering tool has been developed based on the RNA-guided Cas9 nuclease (Gasiunas, Barrangou et al. 2012; Jinek, Chylinski et al. 2012; Cong, Ran et al. 2013; Mali, Yang et al. 2013) from the type II prokaryotic CRISPR (Clustered Regularly Interspaced Short palindromic Repeats) adaptive immune system (see for review (Sorek, Lawrence et al. 2013)). The CRISPR Associated (Cas) system was first discovered in bacteria and functions as a defense against foreign DNA, either viral or plasmid. CRISPR-mediated genome engineering first proceeds by the selection of target sequence which can be flanked by a short sequence motif, referred as the proto-spacer adjacent motif (PAM). Following target sequence selection, a specific crRNA, complementary to this target sequence is engineered. Trans-activating crRNA (tracrRNA) required in the CRISPR type II systems paired to the crRNA and bound to the provided Cas9 protein. Cas9 acts as a molecular anchor facilitating the base pairing of tracRNA with cRNA (Deltcheva, Chylinski et al. 2011). In this ternary complex, the dual tracrRNA:crRNA structure acts as guide RNA (gRNA) that directs the endonuclease Cas9 to the cognate target sequence. Target recognition by the Cas9-tracrRNA:crRNA complex is initiated by scanning the target sequence for homology between the target sequence and the crRNA. In addition to the target sequence-crRNA complementarity, DNA targeting requires the presence of a short motif adjacent to the protospacer (protospacer adjacent motif - PAM). Recently, it has been demonstrated that Cas9 HNH domain is responsible for nicking of one strand of the target double-stranded DNA and the Cas9 RuvC-like RNaseH fold domain is involved in cleavage of the other strand of the double-stranded DNA target (Jinek, Chylinski et al. 2012). Together, these two domains each nick a strand of the target DNA within the proto-spacer in the immediate vicinity of the PAM, which results in blunt cleavage of the invasive DNA (Jinek, Chylinski et al. 2012). The catalytically inactive form of the Cas9 has been used to specifically bind target genes. In particular, Cas9 comprises the two catalytic domains HNH and RuvC inactivated and lacks endonucleolytic activity. The resulting Cas9 co-expressed with gRNA yields to specific targeting of the gene of interest.

In addition to the preceding features, the disclosure comprises further features which will emerge from the following examples illustrating the method of engineering allogeneic and resistant T-cells for immunotherapy, as well as to the appended drawings.

### Example 1-Detection of double strand and single strand DNA using TALE RAGT2-Renila detector system

### Principle

The detection method consists in using a TALE DNA binding domain fused to a Renila Luciferase to detect a single strand DNA (ssDNA) sequence of interest via luminescence emission. It relies on strepavidin coated magnetic beads, a biotinylated ssDNA "bait", complementary to the sequence "pray" to detect and a TALE-Luciferase detector protein (TALE-LUC) specific for the double strand DNA (dsDNA) pray:bait complex (figure 13).

The first step of this method consists in anchoring the ssDNA bait onto the streptavidin coated magnetic beads (figure 14A). This anchoring step is followed by multiple washing steps to remove unattached ssDNA bait. The pool of ssDNA containing the ssDNA pray to detect is then added to magnetic beads (figure 14B, step2). In optimized temperature, buffer and salt conditions, the ssDNA pray is expect to anneal with the ssDNA bait in a highly specific manner. This annealing step enables an efficient and specific capture of the DNA pray among the pool of ssDNA molecules. This first capture is then followed by washing steps to remove non specific ssDNA molecules (mismatched DNA). The TALE-LUC, specifically designed to bind the DNA pray strand of the dsDNA pray:bait complex, is then added to the beads and bind to its target specifically (figure 14C, step 3). This highly specific binding step represents the second capture of the dsDNA to detect. Non specific complex are then washed out and the remaining specific TALE_LUC:dsDNApray:bait complex is eventually detected after addition of luciferase-specific revelation solution and luminometry analysis (figure 14D, step 4). Detection could be multiplexed by using multiple TALE detector protein specifically engineered to emit different signals (Figure 15).

This method is expected to avoid false positive signals due to the formation of TALE-LUC:non specific ssDNA or TALE-LUC:mismatched dsDNA complexes. Indeed, on one hand, false positive complex consisting in TALE-LUC bound to ssDNA bait are unlikely because of two reasons. First, as TALE are known to interact with ssDNA with poor affinity, TALE-LUC:non specific ssDNA complex is expected to be disrupted by subsequent washing steps. Second, TALE-LUC are designed to interact with the sense strand (DNA pray) of the dsDNApray:bait complex. Thus, the TALE-LUC:ssDNA bait is not expected to be formed. On the other hand, false positive complex consisting in TALE-LUC bound to mismatched dsDNA are likely to be avoided for two reasons. First, mismatched dsDNA are unlikely to be formed by annealing under optimized conditions and second, they will poorly bind to TALE-LUC because of the intrinsic specificity of TALE DNA binding domain.

In summary, the ssDNA pray to detect is selected among random ssDNA molecules by two types of captures. The first capture is based on highly specific DNA/DNA interaction while the second one is rather based on TALE/DNA interaction. Both captures are expected to prevent non specific interaction between random ssDNA and TALE-LUC and to promote efficient and specific detection of ssDNA pray. Finally, as TALE DNA binding domains have the ability to bind DNA/RNA heteroduplex, the method 1 could be adapted to detect RNA pray.

We chose to assemble a TALE-LUC consisting in the TALE DNA binding domain targeting RAGT2 target (SEQ ID 25) harboring the conventional deltaN152 terminal domains and a C terminal domain consisting in a C11 truncation fused to a Renila luciferase (pCLS 24992, SEQ ID NO 1). Such system will be called TALE RAGT2-Renila detector system. This construction allowed us to express the corresponding protein in E coli and purify it via Nickel affinity column, taking advantage of the His tag present at the Nterm of the construction. TALE RAGT2-Renila detector system was overexpressed and purified to 80% homogeneity as illustrated in Figure 16 according to the protocol described in Valton et al. (2012).

### - Sensitivity assessment of the TALE RAGT2-Renila detector system

We first sought to assess the TALE RAGT2-Renila detector system sensitivity. To do so, increasing amounts of TALE RAGT2-Renila (from 0 to 10 pmol) were incubated in 96 well plate. Renila reaction mix was then added to each well, the luminescence was measured and plotted as a function of TALE RAGT2-Renila quantity (Figure 17). Our results showed that the luminescence determined in this experiment was proportional to the amount of TALE RAGT2-Renila added to the well from 0.5 to 10 pmol (Figure 17A). Such correlation was lost below 0.05 pmol (Figure 17B). The sensitivity of the detection system is > or = to 0.05 pmol of RAGT2-Renila.

We then evaluated the amount of TALE RAGT2-Renila able to proficiently bind dsDNA immobilized onto streptavidine coated plate (96 well plate format). To do so we immobilized wild type biotinylated dsDNA target (SEQ ID NO 2 and 3), incubated it with increasing amounts of TALE RAGT2-Renila and determined the amount of TALE RAGT2-Renila-DNA complex by luminescence. Briefly, about 13 pmol of biotinylated dsDNA wild type (SEQ ID NO 2 and 3) were immobilized on the streptavidine coated plate. Wells were then washed with high and low salt containing TBST buffers (50 mM Tris buffer, 150 mM NaCL, 0.05% TWEEN 20 and 50 mM Tris buffer, 1 M NaCL, 0.05% TWEEN 20 respectively) and increasing amounts of TALE RAGT2-Renila (from 0 to 750 pmol total) were added to each well. Unbound TALE RAGT2-Renila molecules were then removed by two low salt washing steps and the amount of RAGT2-Renila-DNA complex was determined by luminescence. The luminescence obtained in the presence of increasing amounts of TALE RAGT2-Renila is illustrated on Figure 18A. Thanks to the standard curve obtained in figure 5, the luminescence was converted to the amount of pmol of RAGT2-Renila-DNA complex present in each well (Figure 18B).

Our result showed that 200 pmol of TALE RAGT2-Renila needed to be added to fully saturate about 13 pmol of immobilized dsDNA. Thus our protein preparation contained about 7 % of TALE RAGT2-Renila able to proficiently bind dsDNA. In the experiment described to assess the specificity of the TALE RAGT2-Renila detector system, 300 pmol of TALE RAGT2-Renila will be used systematically (see the next example) to make sure to fully bind the DNA to detect.

After determining the amount of "competent" TALE RAGT2-Renila present in our protein preparation, we investigated its detection sensitivity toward the ssDNA pray. Briefly, decreasing amounts of ssDNA pray wild type (SEQ ID NO 5, from 10 to 0 pmol) were incubated with the ssDNA biotinylated bait (SEQ ID NO 4, 10 pmol) already immobilized on the streptavidine coated plate. The mixture was heated and allowed to cool down to RT. After washing the well with high and low salt containing TBST buffers, an excess of TALE RAGT2-Renila was added to the well (^{∼}300 pmol total), the unbound was then removed by two low salt TBST buffer washing steps and the reaction mixture was added to determine luminescence. The luminescence obtained for each quantity of ssDNA pray used is illustrated in Figure 19A (open circles). Thanks to the standard curve obtained in Figure 17, the luminescence was converted to the amount of pmol of RAGT2-Renila-DNA complex present in each well (Figure 19B, open circle). Our result showed that the of TALE RAGT2-Renila detector system is able to detect > or = to 0.5 pmol of ssDNA pray target (SEQ ID NO 5) with good accuracy. Increasing ssDNA pray quantity in the well, resulted in a proportional increase of Luminescence. Indeed, by plotting the amount of TALE RAGT2-Renila-DNA complex detected (pmol) as a function of ssDNA pray added (pmol), one can perfom a linear regression between the two variable and determine a proportionality ratio close of 1,18 (R=0.995). Thus the TALE RAGT2-Renila detector system is able to detect down to 0.5 pmol of ssDNA pray in a quantitative manner.

To assess the influence of exogenous non specific DNA molecule on the ability of TALE RAGT2-Renila detector system to detect its ssDNA pray (SEQ ID NO 5), the same experiment was performed in the presence of 10 pmol of fragmented DNA plasmid (temperature fragmentation resulting in random DNA fragments of 150 bp length). Increasing ssDNA pray quantity in the well, resulted in a proportional increase of luminescence that was highly similar to the one obtained in the absence of exogenous non specific DNA. Such high similarity was constant whatever the amount of ssDNA pray added in the well as demonstrated by the steadiness of the ratio of luminescence obtained in the absence of exogenous DNA versus the one obtained in the presence of exogenous DNA plasmid (Figure 19A and 19B, lower panels). Altogether, these results demonstrated that TALE RAGT2-Renila was able to detect ssDNA pray, in highly sensitive manner and without being significantly affected by the presence of random DNA molecules. This was particularly striking at low amount of ssDNA pray (0.05 pmol) where the amount of non specific DNA is 200 times higher than the specific ssDNA.

### -Specificity assessment

Different ssDNA targets (SEQ ID NO 6-20) harboring unique or multiple mutations with respect to the ssDNA wild type pray (SEQ ID NO 5, 10 pmol) were synthesized and used to assess the specificity of TALE RAGT2-Renila detector system. We first studied the effect of increasing amount of mutation within the ssDNA target on the luminescence of TALE RAGT2-Renila according to the detection protocol described earlier. Briefly, 10 pmol of ssDNA wild type or mutated were incubated with the ssDNA biotinylated pray already immobilized on the streptavidine coated plate. The mixture was heated and allow to cool down to RT. After washing the well with high and low salt containing TBST buffers, an excess of TALE RAGT2-Renila was added to the well (^{∼}300 pmol total), the unbound was then removed by two low salt buffer washing steps and the reaction mixture was added to determine luminescence. The luminescence obtained for each of the ssDNA prays used is illustrated in Figure 20.

Our results showed that one mutation at the T0 is sufficient to reduce the luminescence signal by more than 60 %. Additional mutations reduced the signal even further indicating that that detector system is sensitive to mono and by extension, multiple mutations present within the ssDNA pray to detect. We then investigated the effect of one single mutation T->C on the luminescence signal using different ssDNA prays (SEQ ID NO 5 and 11-16). Such mutation was intentionally positioned at different locations of the ssDNA pray to study the influence of its position on the luminescence signal alteration (Figure 21). Our results showed that the T to C mutation at the T0 position had a much higher deleterious influence on the luminescence signal than a T to C mutation located at the 3' end of the target. This result fully agreed with the well known positional effect of mismatch described in the literature (Meckler et al, 2013). Such positional effect could also be seen to a greater extent by combining 2 and 3 mutations either at the 5' or 3' end of the ssDNA pray (SEQ ID NO 17-20, Figure 22A and 22B respectively).

### Example 2-Detection of single strand RNA (ssRNA) using TALE RAGT2-Renila detector system

### Principle

The principle of ssRNA pray detection is based on the method delineated above to detect ssDNA pray. It includes all the steps (1-4) described above. However, instead of using a biotinylated DNA bait corresponding to the reversed and complementary sequence of TALE RAGT2 target (SEQ ID NO 4), the biotinylated DNA bait used in the method described herein, corresponds to the actual TALE RAGT2 target (SEQ ID NO 21, Figure 23). Such bait molecule is designed to specifically anneal to the the ssRNA pray to detect (SEQ ID NO 23). Once formed, the DNA/RNA heteroduplex could be potentially recognized by TALE RAGT2-Renila and eventually detected via a luminescence.

### -Experimental proof of concept and specificity assessment

According to the protocol described for the detection of ssDNA pray, ssRNA pray (SEQ ID NO 23, 10 pmol) was incubated with ssDNA biotinylated bait (SEQ ID NO 21) already immobilized on a streptavidin coated 96 well plate. The mixture was heated and allowed to cool down to RT. After washing the well with high and low salt containing buffers, an excess of TALE RAGT2-Renila was added to the well (^{∼}300 pmol total), the unbound was then removed by two low salt washing steps and the reaction mixture was added to determine luminescence. The luminescence obtained for each experiment (wild type ssDNA pray, wild type ssRNA and random ssRNA pray, SEQ ID NO 22, 23 and 24 respectively) are illustrated in Figure 24.

Our results showed that the TALE RAGT2-Renila detector system was able to detect 10 pmol of DNA/RNA heteroduplex to the same extent than a DNA/DNA homoduplex. Such detection was specific to the ssRNA wild type pray (SEQ ID NO 23). Indeed the luminescence signal obtained in the presence of random ssRNA (SEQ ID NO 24) was similar to the one obtained in the negative control performed with the biotinylated bait alone (SEQ ID NO 21). Altogether, our data demonstrate that the TALE RAGT2 detector system is able to detect DNA/RNA heteroduplex in a specific and quantitative manner.

### REFERENCES

Arimondo, P. B., C. J. Thomas, et al. (2006). "Exploring the cellular activity of camptothecin-triple-helix-forming oligonucleotide conjugates." Mol Cell Biol 26(1): 324-33.
Boch, J., H. Scholze, et al. (2009). "Breaking the code of DNA binding specificity of TAL-type III effectors." Science 326(5959): 1509-12.
Bogdanove, A. J. and D. F. Voytas (2011). "TAL effectors: customizable proteins for DNA targeting." Science 333(6051): 1843-6.
Byrum, S. D., S. D. Taverna, et al. (2013). "Purification of a specific native genomic locus for proteomic analysis." Nucleic Acids Res.
Cannata, F., E. Brunet, et al. (2008). "Triplex-forming oligonucleotide-orthophenanthroline conjugates for efficient targeted genome modification." Proc Natl Acad Sci U S A 105(28): 9576-81.
Christian, M., T. Cermak, et al. (2010). "Targeting DNA double-strand breaks with TAL effector nucleases." Genetics 186(2): 757-61.
Christian, M. L., Demorest, Z. L., Starker, C. G., Osborn, M. J., Nyquist, M. D., Zhang, Y., Carlson, D. F., Bradley, P., Bogdanove, A. J., and Voytas, D. F. Targeting G with TAL effectors: a comparison of activities of TALENs constructed with NN and NK repeat variable di-residues. PLoS One 7, e45383
Ciafre, S. A., M. Rinaldi, et al. (1995). "Stability and functional effectiveness of phosphorothioate modified duplex DNA and synthetic 'mini-genes'." Nucleic Acids Res 23(20): 4134-42.
Cong, L., F. A. Ran, et al. (2013). "Multiplex genome engineering using CRISPR/Cas systems." Science 339(6121): 819-23.
Cong, L., R. Zhou, et al. (2012). "Comprehensive interrogation of natural TALE DNA-binding modules and transcriptional repressor domains." Nat Commun 3: 968.
Deltcheva, E., K. Chylinski, et al. (2011). "CRISPR RNA maturation by trans-encoded small RNA and host factor RNase III." Nature 471(7340): 602-7.
Deng, D., Yan, C., Pan, X., Mahfouz, M., Wang, J., Zhu, J. K., Shi, Y., and Yan, N. (2012) Structural basis for sequence-specific recognition of DNA by TAL effectors. Science 335, 720-723
Eisenschmidt, K., T. Lanio, et al. (2005). "Developing a programmed restriction endonuclease for highly specific DNA cleavage." Nucleic Acids Res 33(22): 7039-47.
Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.
Jinek, M., K. Chylinski, et al. (2012). "A programmable dual-RNA-guided DNA endonuclease in adaptive bacterial immunity." Science 337(6096): 816-21.
Lackner, G., N. Moebius, et al. (2011). "Complete genome sequence of Burkholderia rhizoxinica, an Endosymbiont of Rhizopus microsporus." J Bacteriol 193(3): 783-4.
Lackner, G., N. Moebius, et al. (2011). "Evolution of an endofungal lifestyle: Deductions from the Burkholderia rhizoxinica genome." BMC Genomics 12: 210.
Mak, A. N., Bradley, P., Cernadas, R. A., Bogdanove, A. J., and Stoddard, B. L. (2012) The crystal structure of TAL effector PthXo1 bound to its DNA target. Science 335, 716-719
Mali, P., L. Yang, et al. (2013). "RNA-guided human genome engineering via Cas9." Science 339(6121): 823-6.
Meckler JF, Bhakta MS, Kim MS, Ovadia R, Habrian CH, et al. (2013) Quantitative analysis of TALE-DNA interactions suggests polarity effects. Nucleic Acids Res 41: 4118-4128.
Morbitzer, R., J. Elsaesser, et al. (2011). "Assembly of custom TALE-type DNA binding domains by modular cloning." Nucleic Acids Res 39(13): 5790-9.
Morbitzer, R., Romer, P., Boch, J., and Lahaye, T. (2011) Regulation of selected genome loci using de novo-engineered transcription activator-like effector (TALE)-type transcription factors. Proc Natl Acad Sci U S A 107, 21617-21622.
Moscou, M. J. and A. J. Bogdanove (2009). "A simple cipher governs DNA recognition by TAL effectors." Science 326(5959): 1501.
Porteus, M. H. and D. Carroll (2005). "Gene targeting using zinc finger nucleases." Nat Biotechnol 23(8): 967-73.
Simon, P., F. Cannata, et al. (2008). "Sequence-specific DNA cleavage mediated by bipyridine polyamide conjugates." Nucleic Acids Res 36(11): 3531-8.
Sorek, R., C. M. Lawrence, et al. (2013). "CRISPR-mediated Adaptive Immune Systems in Bacteria and Archaea." Annu Rev Biochem.
Valton, J., Dupuy, A., Daboussi, F., Thomas, S., Marechal, A., Macmaster, R., Melliand, K., Juillerat, A., and Duchateau, P. (2012) Overcoming Transcription Activator-like Effector (TALE) DNA Binding Domain Sensitivity to Cytosine Methylation. J Biol Chem 287, 38427-38432
Yin, P., Deng, D., Yan, C., Pan, X., Xi, J. J., Yan, N., and Shi, Y. (2012) Specific DNA-RNA hybrid recognition by TAL effectors. Cell Rep 2, 707-713

## Claims

1. A method of specifically detecting the presence or the absence of a single strand nucleic acid sequence of interest (pray) in nucleic acids, comprising:
(a) providing nucleic acids which can comprise the nucleic acid sequence of interest;
(b) providing a labeled nucleic acid sequence (bait) comprising a complementary sequence to a part of said pray sequence;
(c) hybridizing said pray and bait sequences;
(d) providing at least one DNA binding domain derived from a TALE protein, wherein said DNA binding domain is fused to a reporter molecule and is capable of binding the hybridized pray: bait sequence;
(e) contacting said nucleic acids with said DNA binding domain;
(f) removing unbound said DNA binding domain and nucleic acids and;
(g) detecting the presence or the absence of the nucleic acid sequence of interest in the nucleic acids.

2. The method of claim 1, further comprising quantifying said nucleic acid of interest.

3. The method according any one of claim 1 or claim 2, wherein said nucleic acid of interest is a RNA or a DNA.

4. The method according to any one of claims 1 to 3, wherein said nucleic acids are in a biological sample.

5. The method according to any one of claims 1 to 4, wherein said nucleic acids are within a cell.

6. The method of any one of claims 1 to 5, wherein the reporter molecule is selected from the group consisting of: fluorescent protein, chemiluminescent molecule, tag and enzyme.

7. The method according to any one of claims 1 to 6, wherein the DNA-binding domain and/or said bait sequence are immobilized on a solid support.

## Patentansprüche

1. Verfahren zum spezifischen Detektieren der Anwesenheit oder Abwesenheit einer interessierenden einzelsträngigen Nukleinsäuresequenz (Beute) in Nukleinsäuren, umfassend:
(a) das Bereitstellen von Nukleinsäuren, welche die interessierende Nukleinsäuresequenz umfassen können;
(b) das Bereitstellen einer markierten Nukleinsäuresequenz (Köder), die eine zu einem Teil der Beute-Sequenz komplementäre Sequenz umfasst;
(c) das Hybridisieren der Beute- und Köder-Sequenzen;
(d) das Bereitstellen zumindest einer von einem TALE-Protein abgeleiteten DNA-Bindungsdomäne, wobei die DNA-Bindungsdomäne mit einem Reportermolekül fusioniert ist und geeignet ist, die hybridisierte Beute:Köder-Sequenz zu binden;
(e) das in Kontakt Bringen der Nukleinsäuren mit der DNA-Bindungsdomäne;
(f) das Entfernen ungebundener DNA-Bindungsdomäne und Nukleinsäuren und;
(g) das Detektieren der Anwesenheit oder Abwesenheit der interessierenden Nukleinsäuresequenz in den Nukleinsäuren.

2. Verfahren nach Anspruch 1, zudem umfassend das Quantifizieren der interessierenden Nukleinsäure.

3. Verfahren nach einem der Ansprüche 1 oder 2, wobei die interessierende Nukleinsäure eine RNA oder eine DNA ist.

4. Verfahren nach einem der Ansprüche 1 bis 3, wobei die Nukleinsäuren in einer biologischen Probe sind.

5. Verfahren nach einem der Ansprüche 1 bis 4, wobei die Nukleinsäuren innerhalb einer Zelle sind.

6. Verfahren nach einem der Ansprüche 1 bis 5, wobei das Reportermolekül ausgewählt ist aus der Gruppe bestehend aus: fluoreszierendem Protein, chemilumineszentem Molekül, Tag und Enzym.

7. Verfahren nach einem der Ansprüche 1 bis 6, wobei die DNA-Bindungsdomäne und/oder die Ködersequenz auf einem festen Träger immobilisiert sind.

## Revendications

1. Procédé pour spécifiquement détecter la présence ou l'absence d'une séquence d'acide nucléique simple brin d'intérêt (proie) dans des acides nucléiques, comprenant :
(a) la mise à disposition d'acides nucléiques qui peuvent comprendre la séquence d'acide nucléique d'intérêt ;
(b) la mise à disposition d'une séquence d'acide nucléique marquée (appât) comprenant une séquence complémentaire à une partie de ladite séquence proie ;
(c) l'hybridation desdites séquences proie et appât ;
(d) la mise à disposition d'au moins un domaine de liaison à l'ADN dérivé d'une protéine TALE, où ledit domaine de liaison à l'ADN est fusionné à une molécule rapporteur et est capable de se lier à la séquence proie:appât hybridée ;
(e) la mise en contact desdits acides nucléiques avec ledit domaine de liaison à l'ADN ;
(f) l'élimination desdits domaine de liaison à l'ADN et acides nucléiques non liés et ;
(g) la détection de la présence ou de l'absence de la séquence d'acide nucléique d'intérêt dans les acides nucléiques.

2. Procédé selon la revendication 1, comprenant en outre la quantification dudit acide nucléique d'intérêt.

3. Procédé selon l'une quelconque de la revendication 1 ou la revendication 2, dans lequel ledit acide nucléique d'intérêt est un ARN ou un ADN.

4. Procédé selon l'une quelconque des revendications 1 à 3, dans lequel lesdits acides nucléiques sont dans un échantillon biologique.

5. Procédé selon l'une quelconque des revendications 1 à 4, dans lequel lesdits acides nucléiques sont dans une cellule.

6. Procédé selon l'une quelconque des revendications 1 à 5, dans lequel la molécule rapporteur est sélectionnée dans le groupe consistant en : une protéine fluorescente, une molécule chimiluminescente, un marqueur et une enzyme.

7. Procédé selon l'une quelconque des revendications 1 à 6, dans lequel le domaine de liaison à l'ADN et/ou ladite séquence appât sont immobilisés sur un support solide.
